# EUROPEAN PATENT SPECIFICATION

(11) **EP 1 267 940 B1**
(45) Date of publication and mention of the grant of the patent: **14.05.2003**
(21) Application number: 01921524.3
(22) Date of filing: 28.03.2001
(51) Int. Cl.: A61K 47/48

(54) **COMPOUNDS FOR TARGETING**
VERBINDUNGEN ZUM TARGETING
COMPOSES DE MARQUAGE

(30) Priority: 28.03.2000 GB 0007343
(43) Date of publication of application: 02.01.2003
(73) Proprietor: ANTISOMA RESEARCH LIMITED, London W5 3QR (GB)
(72) Inventor: Epenetos, Agamemnon A., Antisoma Res. Lab., London SW17 0QS (GB)
(74) Representative: Thomas, Philip John Duval
(86) International application number: GB0101354
(87) International publication number: WO01072336

(56) References cited:
- WO-A-92/08495
- WO-A-96/06116

## Description

The present invention relates to compounds, some of which may be indirectly cytotoxic combinations of compounds, which have a high avidity for, and can be targeted to, selected cells.

### Background

The cell-specific targeting of compounds that are directly, or indirectly, cytotoxic has been proposed as a way to combat diseases such as cancer. Bagshawe and his co-workers have disclosed (Bagshawe (1987) *Br. J. Cancer* **56,** 531; Bagshawe *et al* (1988) *Br. J. Cancer* **58,** 700; WO 88/07378) conjugated compounds comprising an antibody or part thereof and an enzyme, the antibody being specific to tumour cell antigens and the enzyme acting to convert an innocuous pro-drug into a cytotoxic compound. The cytotoxic compounds were alkylating agents, *e.g*. a benzoic acid mustard released from *para*-N-bis(2-chloroethyl)aminobenzoyl glutamic acid by the action of *Pseudomonas sp.* CPG2 enzyme.

An alternative system using different pro-drugs has been disclosed (WO 91/11201) by Epenetos and co-workers. The cytotoxic compounds were cyanogenic monosaccharides or disaccharides, such as the plant compound amygdalin, which release cyanide upon the action of a β-glucosidase and hydroxynitrile lyase.

In a further alternative system, the use of antibody-enzyme conjugates containing the enzyme alkaline phosphatase in conjunction with the pro-drug etoposide 4'-phosphate or 7-(2'-aminoethyl phosphate)mitomycin or a combination thereof have been disclosed (EP 0 302 473; Senter *et al* (1988) *Proc. Natl. Acad. Sci. USA* **85,** 4842).

WO-A-9208495 describes TNF, IL-2, lymphotoxin and GM-CSF conjugates inducing apoptosis.

Rybak and co-workers have disclosed (Rybak *et al* (1991) *J. Biol. Chem.* **266**, 21202; WO 91/16069) the cytotoxic potential of a monomeric pancreatic ribonuclease when injected directly into *Xenopus* oocytes and the cytotoxic potential of monomeric RNase coupled to human transferrin or antibodies directed against the transferrin receptor. The monomeric RNase hybrid proteins were cytotoxic to human erythroleukaemia cells *in vitro.*

Other approaches are the *in vivo* application of streptavidin conjugated antibodies followed, after an appropriate period, by radioactive biotin (Hnatowich *et al* (1988) *J. Nucl. Med.* **29,** 1428-1434), or injection of a biotinylated mAb followed by radioactive streptavidin (Paganelli *et al* (1990) *Int. J. Cancer* **45,** 1184-1189). A pilot radioimmunolocalisation study in non-small cell lung carcinomas was conducted with encouraging results (Kalofonos *et al* (1990) *J. Nucl. Med*. **31,** 1791-1796).

Apart from these examples, it is rather more common to see biotinylated antibodies and streptavidin-enzyme conjugates, which are used in enzyme-linked immunosorbent assays.

These previous systems have used relatively large antibody-enzyme, antibody-streptavidin or antibody-biotin conjugates and may comprise portions of non-mammalian origin which are highly immunoreactive.

Rapid penetrance (Yokota *et al* (1992) *Cancer Res.* **52,** 3402-3408) and rapid clearance (Colcher *et al* (1990) *J. Natl. Cancer Inst.* **82,** 1191-1197) have been demonstrated for single chain Fv antibody fragments (ScFv).

In using the cell-specific reagents aforementioned in a therapeutically useful situation, one of the requirements that needs to be met is for the cell-specific reagent to accumulate to a sufficiently higher level at the target cell than at other cells. A further requirement is that a directly or indirectly cytotoxic reagent is carried to the target cell, and it is preferred that the said cytotoxic reagent is of high potency.

We have now devised improved systems, at least some of which make use of novel, potent indirectly-cytotoxic agents.

### Summary of Invention

A first aspect of the invention provides a compound comprising a target cell-specific portion and a cytotoxic portion characterised in that the cytotoxic portion is a constitutively active caspase or has substantially the same apoptosis-inducing activity as the said caspases.

Apoptosis, or 'programmed cell death', is a highly orchestrated and evolutionarily conserved form of cell death in which cells deliberately destroy themselves. Unlike necrosis, where the cell is killed often as the result of chemical or toxic attack, during apoptosis the cell intentionally dismantles itself in a systematic manner, leading to the production of membrane-packaged sub-cellular fragments ('apoptotic bodies').

Apoptosis is critical to the health of multicellular organisms since mammalian cells are not intended to be immortal. For example, the process is vital to sculpt the nervous system during development and to maintain the normal functioning of the immune system. Dysfunction of apoptotic mechanisms can lead to disease. For example, when there is too little apoptosis, cancer or autoimmune disease may occur, when there is too much apoptosis, stroke damage or neurodegeneration may occur (see review by Reed (1999) ***J.** Clin. Oncol.* **17,** 2941-2953; Wolf & Green (1999) *J. Biol. Chem.* **274,** 20049-20052).

The signalling cascade that results in apoptosis is complex and diverse. 'Death receptors' exist on the cell surface *(e.g.* Fas or TRAIL receptors) which, when activated, transmit a triggering signal resulting in the apoptotic events (Ashkenazi & Dixit (1998) *Science* **281,** 1305-1308). In addition, insults which damage DNA, such as UV or X-ray irradiation, chemotherapeutic drugs and viral infection, can be detected by the cell and trigger off cell death if repair is unfeasible (Benjamin, Hiebsch & Jones (1998) *Mol. Pharmacol*. **53,** 446-450).

Initial studies in the nematode, *Caenorhabditis elegans,* revealed three genes essential for apoptosis; CED-3 and CED-4 (which promote apoptosis), and CED-9 (which inhibits apoptosis). CED-3 encodes a cysteine-bearing aspartate protease (or 'caspase'), that is activated by CED-4 and inactivated by CED-9. Subsequent to these initial studies, mammalian counterparts for most of the *C. elegans* genes have been discovered, including a range of caspases related to CED-3.

Central to the execution of apoptosis is a proteolytic cascade involving the family of caspase proteases. Each proteolytic step is irreversible, leading to the final activation of a number of events that result in the morphological characteristics of apoptosis (Reed (1999) ***J.*** *Clin. Oncol.* **17,** 2941-2953; Wolf & Green (1999) *J. Biol. Chem*. **274,** 20049-20052).

During the execution of apoptosis, initiator caspases activate effector caspases, which in turn trigger many downstream events that ultimately lead to apoptotic cell death (Reed (1999) ***J.*** *Clin. Oncol.* **17,** 2941-2953; Wolf & Green (1999) *J. Biol. Chem.* **274,** 20049-20052). The first targets to be cleaved are believed to be pro-apoptotic proteins (*e*.*g*. other caspases), which amplify the signal for cell death and reduce activity of anti-apoptotic proteins (such as Bcl-2/XL, Bid), thereby reducing inhibition of apoptosis. As apoptosis progresses, components such as CAD (Caspase Activated Deoxyribonuclease) (Enari, Sakahira, Yokoyama *et. al*. (1998) *Nature* **391,** 43-50), gelsolin, PAK2, MEKKI and PKCd are produced, which contribute directly to the apoptosis phenotype by inducing chromosomal DNA fragmentation and cytoskeleton disruption. Caspases also cleave structural proteins such as lamins, actin, keratins, fodrin, Gas2, NuMa and SAF-A, promoting nuclear dissolution and cytoskeletal breakdown. Other substrates for caspase cleavage include homeostatic proteins such as DNA-PKcs, PARP, U1-70KDa and transcription factors, which are involved in preventing cell repair and macromolecular synthesis.

These events essentially shut down the cell, remove contact with neighbouring cells, re-organise the interior for break-up and signal to the immune system that the cell is marked for phagocytosis after its break-up into apoptotic bodies.

The original CED-3 caspase is related to the mammalian interleukin-1β-converting enzyme, termed caspase-1 or ICE. This led to the discovery of other homologous enzymes, some of which are intimately involved in the apoptotic cascade. All such homologues are pro-enzymes of 30-50 kDa, which are activated to form heterodimers that themselves dimerise to form tetramers (Wilson, Black, Thomson *et. al*. (1994) *Nature* **370,** 270-274; Rotonda, Nicholson, Fazil *et. al*. (1996) *Nat. Struct. Biol*. ***3,*** 619-25). The caspase homologues all recognise a 4-amino acid peptide sequence and cleave after the aspartic acid (see below) (Thombeny, Rano, Peterson *et. al*. (1997) ***J.*** *Biol. Chem*. **272,** 17907-17911).

Caspases exist initially in the form of inactive pro-enzymes, which are constitutively expressed in living cells and are regulated in a complex manner, enabling them to trigger a rapid pathway resulting in cell destruction. The inter-relationship between the activities of the various caspases in this apoptotic pathway is complex. Generally, initiator caspases are activated, leading in turn to activation of effector caspases (Table 1), which mediate the destructive events (Reed (1999) ***J.** Clin. Oncol*. **17,** 2941-2953; Wolf & Green (1999) *J. Biol. Chem*. **274,** 20049-20052). The initiator caspase-8 is triggered via the death receptors, whereas the initiator caspase-9 is triggered by cytotoxic agents. Caspase-8 activates caspase-3 and 7, while caspase-3 may activate caspase-6. Caspase-3 and -6 go on to effect nuclear apoptotic events. Caspase-9 also activates caspases -3 and -7.

Once activated, caspase-3 and -7 (and possibly -6), represent an irreversible commitment to cell death. In contrast, activation of initiation caspases can still be blocked by anti-apoptotic events. The role of other caspases, such as caspases -2, -4, -10 and -13, is less clearly understood.

**Table 1**

| **Name** | **Alternative name** | **Type of caspase** | **Molecular weight of large/small subunits (KDa)** | **Preferred tetrapeptide sequence** |
|---|---|---|---|---|
| Caspase-1¹ | ICE/Ced | Cytokine processing | 20/10 | (W/Y/F)-E-H-D |
| Caspase-2 ² | | Initiator | 20/12 | D-X-X-D |
| Caspase-3 ³ | CPP-32 | Effector/ executioner | 17/12 | D-E-X-D |
| Caspase-4 ⁴ | | Cytokine processing | 20/10 | (W/Y/F)-E-H-D |
| Caspase-5 ⁵ | | Cytokine processing | 20/10 | (W/Y/F)-E-H-D |
| Caspase-6 ⁶ | Mch-2 | Effector/ executioner | 18/11 | (V/T/I)-E-X-D |
| Caspase-7 ⁷ | CMH-1 | Effector/ executioner | 20/12 | D-E-X-D |
| Caspase-8 ⁸ | | Initiator | 18/11 | (L/V/D)-E-X-D |
| Caspase-9 ⁹ | Mch6 | Initiator | 17/10 | (I/VIL)-E-H-D |
| Caspase-10¹⁰ | | Initiator | 17/12 | unknown |
| Caspase-11 ¹¹ | | Cytokine processing | 20/10 | unknown |
| Caspase-12 ¹² | | Cytoldne processing | 20/10 | unknown |
| Caspase-13 ¹³ | | Cytokine processing | 20/10 | unknown |
| Caspase-14¹⁴ | | Cytokine processing | 20/10 | unknown |

| | | | | |
|---|---|---|---|---|
| *Key to Table 1* ¹ Thornberry *et al.* (1992) *Nature* **356**:766-774 | | | | |
| ² Li *et al*. (1997) *J. Biol. Chem.* **272**:21010-7 | | | | |
| ³ Alnemri *et al.* (1996) *Cell* **87**:171 | | | | |
| ⁴ Kamada *et al.* (1997) *Oncogene* **15**:285-90 | | | | |
| ⁵ Kamada *et al*. (1997) *Cell Death and Differentiation* **4(6)**:473-478 | | | | |
| ⁶ LeBlanc *et al.* (1999) *J. Biol. Chem*. **274**:23426-36 | | | | |
| ⁷ Marcelli *et al.* (1998) *Cancer Res.* **58**:76-83 | | | | |
| ⁸ Scaffidi *et al.* (1997) *J. Biol. Chem.* **272**:26953-8 | | | | |
| ⁹ Srinivasula *et al*. (1996) *J. Biol. Chem.* **271**:27099-106 | | | | |
| ¹⁰ Ng *et al*. (1999) *J. Biol. Chem.* **274:**10301-8 | | | | |
| ¹¹ Wang *et al.* (1998) *Cell* **92:**501-9 | | | | |
| ¹² Nakagawa *et al*. (2000) *Nature* **403:**98-103 | | | | |
| ¹³ Humke *et al.* (1998) *J. Biol. Chem.* **273:**15702-7 | | | | |
| ¹⁴ Hu *et al*. (1998) *J. Biol. Chem.* **273:**29648-53 | | | | |

Table 1 shows the properties of several known caspases.

All caspases share a common structure. The caspase pro-enzyme contains an N-terminal pro-domain, a large subunit containing the active site cysteine (within a conserved QACXG motif) and a small subunit which also contributes residues to the inter-subunit active site (Wilson, Black, Thomson *et. al.* (1994) *Nature* **370,** 270-274; Rotonda, Nicholson, Fazil *et al*. (1996) *Nat. Struct. Biol*. **3,** 619-25; Thornberry, Rano, Peterson *et al*. (1997) ***J.*** *Biol. Chem*. **272,** 17907-17911). Upon auto-activation or activation by other caspases, the pro-domain is cleaved at an aspartate residue and an interdomain linker is cleaved at one or two aspartate residues, thereby converting the single-chain enzyme into a enzymatically-active heteroduner (comprising a large subunit and a small subunit). The heterodimer can subsequently dimerise into a tetramer.

Each caspase active site has a conserved positively charged 'S1 pocket', to bind the negatively charged aspartate 'P1' residue in the substrate peptide. Thus, all caspases cleave solely after aspartate residues. However, members of the caspase family exhibit different tetrameric peptide substrate specificities (see Table 1).

The upstream (or initiator) caspases, *e.g*. caspases-2, -8, -9 and-10, have a large pro-domain, which is thought to be involved in the recruitment of such caspases by 'death receptors' (see Cohen, 1997, *Biochem*. **326**:1-16; Kischkel et al., 1995, *EMBO J*. **14:**5579-5588). Association of the initiator receptors with the death complexes results in these enzymes being brought into close proximity with each other, which is thought to facilitate their activation by autocatalytic processing (Yang *et al*., 1988, *Mol. Cell.* 1:319-325; Muzio *et al.,* 1998, *J. Biol. Chem*. **273:**2926-2930).

In contrast, the effector caspases such as caspase-3, -6 and -7 have shorter pro-domains and are not recruited by the death receptors. As a result, these caspases remain dormant until activated directly by the initiator caspases via proteolytic cleavage (Fernandez-Alnemri *et al*., 1996, *Proc. Natl. Acad. Sci. USA* **93**:7464-7469; Srinivasula et al., 1996, *Proc. Natl. Acad. Sci. USA* **93**:13706-13711). Once activated, the effector caspases rapidly dismantle important structural and regulatory components of the cell, leading to the characteristic apoptotic phenotype observed in cells undergoing apoptosis (Nicholson and Thomberry, 1997, *Trends Biochem. Sci.* **257:**299-306).

By "a constitutively active caspase" we include a protein or peptide which exhibits cysteine-bearing aspartate protease activity sufficient to induce apoptosis, i.e. a caspase in an activated form. Alternatively, the constitutively active caspase may comprise a precursor of such an active caspase that is able to *spontaneously* self-catalyse its conversion to the active caspase.

It will be appreciated that the cytotoxic portion comprising a constitutively active caspase or having substantially the same apoptosis-inducing activity as the said caspases may act at any point in the apoptotic cascade of events (as described above). For example, the cytotoxic portion may comprise a constitutively active variant of an effector caspase, such as caspase-3, caspase-6 or caspase-7, which directly triggers downstream apoptotic events. Alternatively, the cytotoxic portion may comprise a constitutively active variant of an initiator caspase such as caspase-2, caspase-8, caspase-9 or caspase-10 which acts upon the effector caspases to induce apoptosis.

Where the cytotoxic portion is a variant of a naturally-occurring caspase, said variant is constitutively active such that it is spontaneously active without the need for activation by other components of the apoptotic cascade.

Exemplary naturally occurring caspases are described in Table 1.

By "a variant" we include cytotoxic portions comprising of a naturally occurring caspase wherein there have been amino acid insertions, deletions or substitutions, either conservative or non-conservative, such that the changes obviate the need for processing of the naturally occurring caspase precursor, and do not substantially reduce the apoptosis-inducing activity of the variant compared to the apoptosis-inducing activity of the activated naturally occurring caspase. For example, the variant may have increased apoptosis-inducing activity compared to the apoptosis-inducing activity of the naturally occurring caspase.

Such variants may be made using methods of protein engineering and site-directed mutagenesis commonly known in the art (*e.g.* US 4,302,386).

Exemplary constitutively active caspases are described in Srinivasula *et al.* (1998) *J. Biol. Chem*. **273**:10107-10111. Said caspases are variants of naturally occurring caspases-3 and -6 wherein the small and large subunits are re-arranged using recombinant techniques. Specifically, the subunit positions are reversed such that the C-terminus of the large subunit and the N-terminus of the small subunit are free, while the N-terminus of the large subunit and the C-terminus of the small subunit are physically linked.
This rearrangement of the subunits allows an active site to be formed by the spontaneous folding and interdigitating of the free C-terminus of the large subunit and N-terminus of the small subunit. In contrast, in the naturally occurring caspase precursor, these ends are contiguous and hence are unable to form an active site until released by cleavage of the precursor polypeptide.

Preferably, the cytotoxic portion is a constitutively active effector caspase or has substantially the same apoptosis-inducing activity as the said caspases.

More preferably, the cytotoxic portion is a constitutively active effector caspase selected from the group consisting of caspase-3, caspase-6 and caspase-7, or has substantially the same apoptosis-inducing activity as the said caspases.

It will be appreciated that the cytotoxic portion may comprise a fragment of a constitutively active caspase, wherein the fragment comprises at least the catalytically active portion of said constitutively active caspase. Thus, the cytotoxic portion may comprise a peptide or protein fragment which substantially retains the apoptosis-inducing activity of a constitutively active caspase, *e.g.* the cytotoxic portion may comprise the active site of constitutively active caspase.
Advantageously, the cytotoxic portion is of mammalian, preferably human, origin. The use of the said mammalian proteins as the directly or indirectly cytotoxic portion of the compound of the invention is advantageous since such compounds are less likely to give rise to undesirable immune reactions.

Preferably, the cytotoxic portion of the compound of the invention is capable of oligomerisation, *e.g.* dimerisation. Attachment of the target-cell specific portion to a cytotoxic portion capable of oligomerisation provides a method for increasing the number of binding sites to the target cell. For example, if the target cell-specific portion is joined to a portion capable of forming a dimer then the number of target cell-specific binding sites is two; if the target cell-specific portion is joined to a portion capable of forming a tetramer then the number of target cell-specific binding sites is four. The number of target cell-specific binding sites is greater than one and the compounds may therefore have a greater avidity for the target cell than do compounds which only have one target cell-specific binding site.

It is preferable for the cytotoxic portion of the compound of the invention capable of oligomerisation to contain no interchain disulphide bonds nor intrachain disulphide bonds; to be well characterised; to be non-toxic; to be stable; to be amenable to preparation in a form suitable for pre-clinical or clinical use or be in pre-clinical or clinical use; and for the subunit monomers to have a high affinity for each other, that is they contain one or more subunit binding sites.

Skilled persons will appreciate that caspase activity may be detected and/or measured by one or more of the following tests:
(i) Assays based on the hydrolysis of D-E-V-D analogue-type substrates that are cleaved to produce a product measurable by colourimetric means (such assays will provide values for binding and catalytic rate constants, Kₘ and k_{cat}, respectively) (for example, see Gurtu *et al.,* 1997, *Anal. Biochem*. **251:**98-102);
(ii) Assays involving physiological observation of cellular apoptosis, *e.g.* using time-lapsed video microscopy, wherein apoptosis is characterised by membrane blebbing and cell shrinkage (for example, see Loke *et al*., 1999, *Eur. J. Immunol*. **29:**1793-802; Weisser *et al*., 1998, *Cytometry* **31:**20-28; Ohta *et al*., 1998, *Brain Tumor Pathol.* **15:**19-21); and
(iii) Assays for products of apoptosis, such as the TUNEL assay, wherein the amount of degraded DNA or antibodies against degraded substrates of apoptotic cleavage *(e.g.* PARP) is measured (Vakkala *et al*., 1999, *Br. J. Cancer* **81:**592-599; Namura *et al.,* 1998, *J. Neurosci*. **18**:3659-3668).

By "target cell specific" portion we mean the portion of the compound which comprises one or more binding sites which recognise and bind to entities on the target cell. Upon contact with the target cell, the target cell specific portion is internalised along with the cytotoxic portion. Such internalisation results in the cytotoxic portion being delivered to the cell cytosol, where it has access to the numerous caspase substrates which are involved in the apoptotic cascade.

The entities recognised by the target cell-specific portion are expressed predominantly, and preferably exclusively, on the said target cell. The target cell specific portion may contain one or more binding sites for different entities expressed on the same target cell type, or one or more binding sites for different entities expressed on two or more different target cell types.

Preferably, the target cell-specific portion recognises the target cell with high avidity.

By "high avidity" we mean that the target cell-specific portion recognises the target cell with a binding constant of at least K_{d} = 10⁻⁶ M, preferably or least K_{d} = 10⁻⁹ M, suitably K_{d} = 10⁻¹⁰ M, more suitably K_{d} = 10⁻¹¹ M, yet more suitably still K_{d} = 10⁻¹² M, and more preferably K_{d} = 10⁻¹⁵M or even K_{d} = 10⁻¹⁸ M.

The entity which is recognised may be any suitable entity which is expressed by tumour cells, virally-infected cells, pathogenic microorganisms, cells introduced as part of gene therapy or even normal cells of the body which, for whatever reason, one wishes to target, but which is not expressed, or at least not with such frequency, in cells which one does not wish to target. The entity which is recognised will often be an antigen. Examples of antigens include those listed in Table 2.

**Table 2**

| 1. ***Tumour Associated Antigens*** | | |
|---|---|---|
| Antigen | Antibody | Existing Uses |
| Carcino-embryonic Antigen | C46 (Amersham) 85A12 (Unipath) | Imaging & Therapy of colon/rectum tumours. |
| Placental Alkaline Phosphatase | H17E2 (ICRF, Travers & Bodmer) | Imaging & Therapy of testicular and ovarian cancers. |
| Pan Carcinoma | NR-LU-10 (NeoRx Corporation) | Imaging & Therapy of various carcinomas |
| | | incl. small cell lung cancer. |
| Polymorphic Epithelial Mucin (Human milk fat globule | HMFG1 (Taylor-Papadimitriou, ICRF) (Antisoma plc) | Imaging & Therapy of ovarian cancer, pleural effusions, breast, lung & other common epithelial cancers. |
| Human milk mucin core protein | SM-3(IgG1)¹ | Diagnosis, Imaging & Therapy of breast cancer |
| β-human Chorionic Gonadotropin | W14 | Targeting of enzyme (CPG2) to human xenograft choriocarcinoma in nude mice. (Searle *et al* (1981) *Br. J. Cancer* **44,** 137-144) |
| A Carbohydrate on Human Carcinomas | L6 (IgG2a)² | Targeting of alkaline phosphatase. (Senter *et al* (1988) *Proc. Natl. Acad. Sci. USA* **85,** 4842-4846 |
| CD20 Antigen on B Lymphoma (normal and neoplastic) | 1F5 (IgG2a)³ | Targeting of alkaline phosphatase. (Senter *et al* (1988) *Proc. Natl. Acad. Sci. USA* **85,** 4842-4846 |

| | | |
|---|---|---|
| ¹Burchell *et al* (1987) *Cancer Res*. **47,** *5476-5482* | | |
| ²Hellström *et al* (1986) *Cancer Res.* **46,** 3917-3923 | | |
| ³Clarke *et al* (1985) *Proc. Natl. Acad. Sci. USA* **82,** 1766-1770 | | |

Other antigens include alphafoetoprotein, Ca-125, prostate specific antigen and members of the epidermal growth factor receptor family, namely EGFR, erb B2 (*e.g.* Herceptin antibody), erb B3 and erb B4.

| 2. ***Immune Cell Antigens*** | | |
|---|---|---|
| Antigen | Antibody | Existing Uses |
| B-lymphocyte Surface Antigen (CD22) | RFB4 (Janossy, Royal Free Hospital) | Immunotoxin therapy of B cell lymphoma. |
| Pan T lymphocyte Surface Antigen (CD5) | H65 (Bodmer, Knowles ICRF, Licensed to Xoma Corp., USA) | Immunotoxin treatment of Acute Graft versus Host disease, Rheumatoid Arthritis. |

| 3. ***Infectious Agent-Related Antigens*** | | |
|---|---|---|
| Antigen | Antibody | Existing Uses |
| Hepatitis B Surface Antigen | Anti HBs Ag | Immunotoxin against Hepatoma. |

In a preferred embodiment, the target cell specific portion recognises and selectively binds to a tumour cell antigen.

Advantageously, the target cell specific portion is an antibody or antigen binding fragment thereof.

Conveniently, the target cell-specific portion comprises two or more binding sites for the target cell, wherein the target cell-specific portion is an antibody, or bivalent fragment thereof. Said target cell-specific portion may have respective 'arms' that recognise the same entity as one another or that recognise different entities.

In one embodiment of the compounds of the invention, the target cell-specific portion has two 'arms' which recognise different molecules on the same target cell wherein the molecules on the same target cell are not confined to that cell type but may occur on a few other cell types. For example, one 'arm' of the target cell-specific portion may recognise molecules on cell types I, II and III, whereas the other 'arm' may recognise molecules on cell types I, IV and V. Thus a compound of the invention comprising such a target cell-specific portion will have greater specificity for cell type I compared with cell types II, III and IV. This aspect of the invention is particularly helpful, as there have been very few completely target cell-specific molecules discovered, whereas molecules which occur on a few cell types, and which are useful in this aspect of the invention, are well known. Such molecules are usually cell-surface antigens for which cross-reactive antibodies are known. Examples of such molecules are given in Table 3.

**Table 3**

| Antigen | Cell-type | Antibody |
|---|---|---|
| CD9 | Pre-B cells, monocytes, platelets | MM2/57 (IgG2b, mouse) |
| CALLA | Lymphoid progenitor cells, granulocytes | B-E3 (IgG2a, mouse) |
| CD13 | Myeloid monocytes, granulocytes | B-F10 (IgG1, mouse) |
| CD24 | B-cells, granulocytes | ALB-9 (IgG1, mouse) |
| CD61 | Platelets, megakaryocytes | PM 6/13 (IgG1, mouse) |

The antibodies described in Table 3 are generally available from Serotec, Oxford, OX5 1BR, UK.

Monoclonal antibodies which will bind to many of the antigens listed in Table 2 are already known, but in any case, with today's techniques in relation to monoclonal antibody technology, antibodies can be prepared to most antigens. The antigen-specific portion may be a whole antibody, a part of an antibody (for example a Fab or F(ab')₂ fragment), a synthetic antibody fragment (for example a single chain Fv fragment [ScFv]), or a peptide/peptidomimetic or similar. Suitable monoclonal antibodies to selected antigens may be prepared by known techniques, for example those disclosed in *"Monoclonal Antibodies: A manual of techniques"*, *H* Zola (CRC Press, 1988) and in *"Monoclonal Hybridoma Antibodies: Techniques and Applications"*, J *G* R Hurrell (CRC Press, 1982) and *Antibody Engineering, A Practical Approach,* McCafferty, J. et al, ed. (IRL Pres, 1996).

The variable heavy (V_{H}) and variable light (V_{L}) domains of the antibody are involved in antigen recognition, a fact first recognised by early protease digestion experiments. Further confirmation was found by "humanisation" of rodent antibodies. Variable domains of rodent origin may be fused to constant domains of human origin such that the resultant antibody retains the antigenic specificity of the rodent parented antibody (Morrison *et al* (1984) *Proc. Natl. Acad. Sci. USA* **81,** 6851-6855).

That antigenic specificity is conferred by variable domains and is independent of the constant domains is known from experiments involving the bacterial expression of antibody fragments, all containing one or more variable domains. These molecules include Fab-like molecules (Better *et* *al* (1988) *Science* **240,** 1041); Fv molecules (Skerra *et al* (1988) *Science* **240,** 1038); disulphide-linked Fv molecules (Young *et al*., 1995, *FEBS Lett*. **377**:135-139); *single-chain* Fv (ScFv) molecules where the V_{H} and V_{L} partner domains are linked via a flexible oligopeptide (Bird *et al* (1988) *Science* **242,** 423; Huston *et al* (1988) *Proc. Natl. Acad. Sci. USA* **85,** 5879) and single domain antibodies (dAbs) comprising isolated V domains (Ward *et al* (1989) *Nature* **341,** 544). A general review of the techniques involved in the synthesis of antibody fragments which retain their specific binding sites is to be found in Winter & Milstein (1991) *Nature* **349,** 293-299.

By "ScFv molecules" we mean molecules wherein the V_{H} and V_{L} partner domains are linked via a flexible oligopeptide.

Chimaeric antibodies are discussed by Neuberger *et al* (1988, *8th International Biotechnology Symposium* Part 2, 792-799).

Suitably prepared non-human antibodies can be "humanised" in known ways, for example by inserting the CDR regions of mouse antibodies into the framework of human antibodies.

The advantages of using antibody fragments, rather than whole antibodies, are several-fold. The smaller size of the fragments allows for rapid clearance, and may lead to improved tumour to non-tumour ratios. Fab, Fv, ScFv, disulphide Fv and dAb antibody fragments can all be expressed in and secreted from bacteria, such as *E. coli,* or eukaryotic expression systems such as Yeast or mammalian systems, thus allowing the facile production of large amounts of the said fragments.

Whole antibodies, and F(ab')₂ fragments are "bivalent". By "bivalent" we mean that the said antibodies and F(ab')₂ fragments have two antigen combining sites. In contrast, Fab, Fv, ScFv, disulphide Fv and dAb fragments are monovalent, having only one antigen combining site.

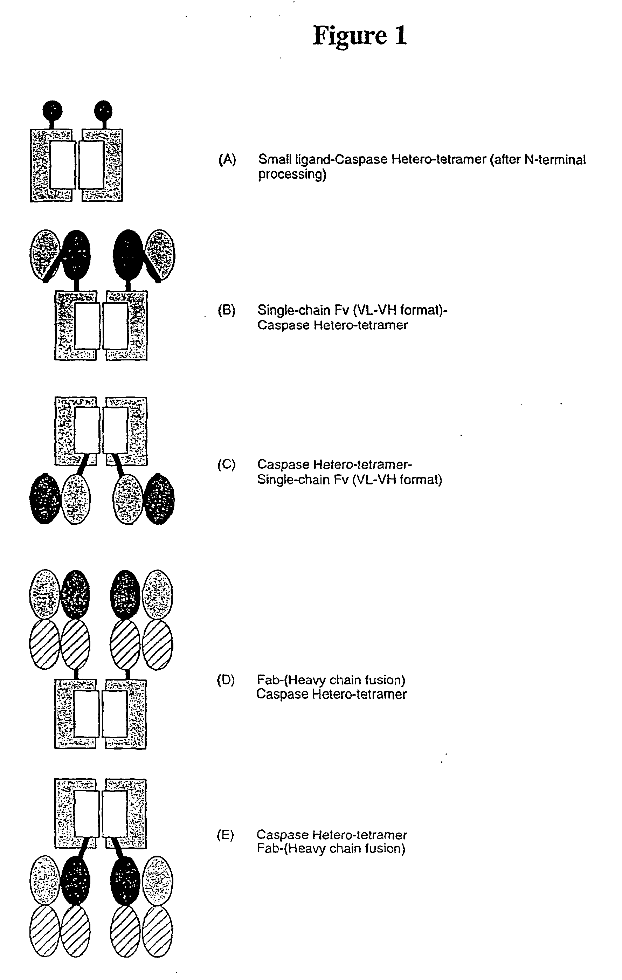

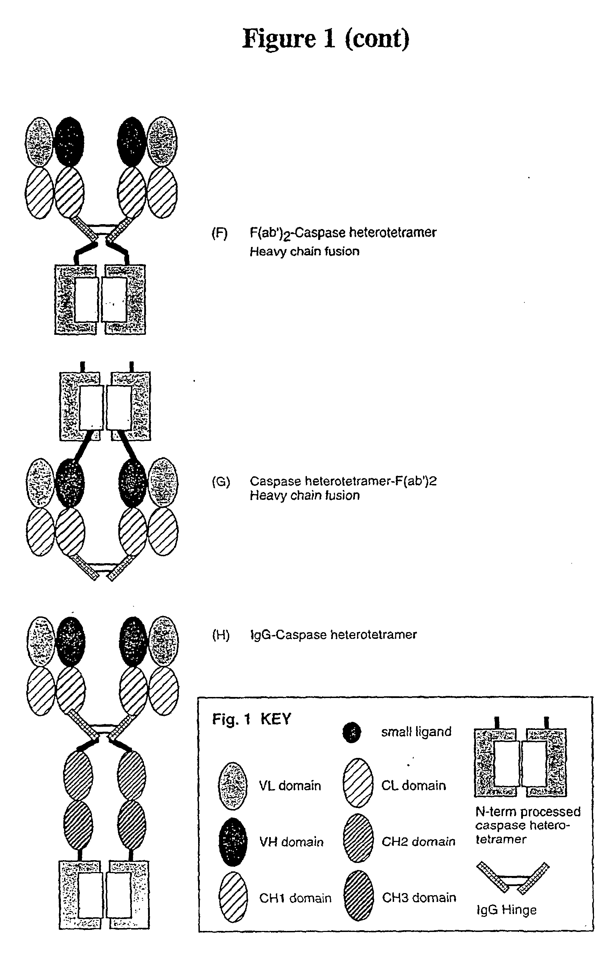

Some of the various compounds of the invention are illustrated diagrammatically in Figure 1. Of course, the cytotoxic portion may form higher order oligomers than those illustrated, for example trimers, tetramers, pentamers, hexamers.

The entity that is recognised may or may not be antigenic but can be recognised and selectively bound to in some other way. For example, it may be a characteristic cell surface receptor such as the receptor for melanocyte-stimulating hormone (MSH) which is expressed in high number in melanoma cells. Alternatively, the entity may be an entity that is induced in the target cells, *e.g.* vascular endothelial growth factor (VEGF) receptors in angiogenesis. The cell-specific portion may then be a compound or part thereof which specifically binds to the entity in a non-immune sense, for example as a substrate or analogue thereof for a cell-surface enzyme or as a messenger.

Preferably, the high avidity target cell specific portion comprises two or more different binding sites for the target cell.

The different binding sites for the target cell may or may not be two or more different antibodies, or fragments thereof, which are directed to different entities expressed on the target cell. Alternatively, the different binding sites for the target cell may recognise and selectively bind the cell in some other, non-immune sense.

A further alternative is that one or more of the binding sites is an antibody, or part thereof, and that one or more of the binding sites for the target cell recognise and selectively bind the cell in some other, non-immune sense.

A compound which has binding sites for two or more target cell-specific entities may be more specific for binding to the said target cell, and a compound which has more than one of each of the different binding sites may bind to the said target cell with greater avidity. In combining two or more binding sites, which in themselves may be of high specificity but low affinity, it will be possible to generate in the compound of the invention a higher affinity for the target cell whilst retaining the specificity of the binding sites.

Many target cell-specific molecules are known, such as those disclosed in Table 2, which are not joined to a further directly or indirectly cytotoxic portion, but may nevertheless be useful in directing cytotoxic agents to a target cell.

Thus, a further aspect of the invention provides a compound comprising a mediator portion and a cytotoxic portion characterised in that the cytotoxic portion is a constitutively active caspase or has substantially the same apoptosis-inducing activity as the said caspases. The cytotoxic portion may comprise any of the cytotoxic portions described above in relation to compounds of the first aspect of the invention.

By "mediator portion" we mean the portion of the compound that recognises a target cell-specific molecule and mediates the internalisation of the cytotoxic portion to the cytosol of the target cell. The target cell-specific molecule may be a compound according to the first aspect of the present invention, or it may be a target cell-specific molecule known in the art, or it may be a derivative thereof capable of recognition by the mediator portion.

In the case of native target cell-specific molecule comprising antibodies, said target cell-specific molecule may be recognised by the mediator via its Fc portion.

The mediator may recognise the native target cell-specific molecule, but it is preferable for the mediator to recognise a derivative of the said molecule.

The said derivative may be made by joining a moiety, such as a small molecule, for example a hapten, to the said molecule. The mediator, for example an antibody or fragment thereof, may then recognise either said hapten or some other part of the derivatised target cell specific molecule.

The advantage in using this method is that the same moiety (*e.g*. hapten) may be joined to all types of target cell-specific molecules, and then only one compound, comprising a mediator which recognises the said moiety and a cytotoxic portion, may be used to deliver the cytotoxic agent to the target cell.

In one embodiment of the invention the mediator comprises an HMFG-1 antibody or antigen binding fragment thereof (*e.g.* scFv), and the moiety recognised by the said HMFG-1 antibody is the hapten, polymorphic epithelial mucin (PEM) core peptide. The target cell-specific molecule is a further antibody comprising said hapten, and specifically comprising a peptide of sequence APDTR within the twenty amino acid tandem repeats of the *muc-1* gene product which is the antigenic determinant of PEM recognised by HMFG-1 antibodies. HMFG-1 antibodies are disclosed in EP 483 961 A.

Before such molecules can be regarded as suitable candidates, there is a requirement that cell specificity be demonstrated and a further requirement that this specificity be shown to be conferred only by the combination of the interaction of the primary targeting antibody with target, and the interaction of the second step reagent (in this case the HMFG-1 antibody) with the primary antibody. To this end, the primary antibody needs to be recognised specifically by the mediator, and therefore requires stable modifications that will distinguish it from native antibodies. Multiple derivatisation of the primary antibody with a hapten fulfils this demand, and has the further advantage of amplification, providing an array of secondary targets for the mediator.

Of course, other mediators such as Fab, F(ab')₂, dAbs, scFv, disuphide-linked Fv or other antibody fragments may be used. Moreover, the mediator may also recognise the moiety in a non-immune sense, such as in biotin-streptavidin recognition. It is preferred if the moiety recognised is a small molecule, but the moiety may also be a polypeptide, peptide, oligosaccharide or the like.

In a preferred aspect of the first and second aspects of the invention, the compound is a fusion compound comprising a target cell-specific portion or mediator portion and a cytotoxic portion characterised in that the cytotoxic portion is a constitutively active caspase or has substantially the same apoptosis-inducing activity as the said caspases.

By "fusion compound" we include a compound comprising one or more functionally distinct portions, wherein the distinct portions are contained within a single polypeptide chain produced by recombinant DNA techniques.

Alternatively, the target-cell specific or mediator portions and the cytotoxic portion of the compound of the invention are separate moieties linked together by any of the conventional ways of cross-linking polypeptides, such as those generally described in O'Sullivan *et al Anal. Biochem*. (1979) **100,** 100-108. For example, the antibody portion may be enriched with thiol groups and the enzyme portion reacted with a bifunctional agent capable of reacting with those thiol groups, for example the N-hydroxysuccinimide ester of iodoacetic acid (NHIA) or N-succinimidyl-3-(2-pyridyldithio)propionate (SPDP). Amide and thioether bonds, for example achieved with m-maleimidobenzoyl-N-hydroxysuccinimide ester, are generally more stable *in vivo* than disulphide bonds.

A third aspect of the invention provides an isolated nucleic acid molecule encoding a compound according to the first or second aspect of the invention, or a target cell specific portion, mediator portion or cytotoxic portion thereof.

By "nucleic acid molecule" we include DNA, cDNA and mRNA molecules.

A fourth aspect of the present invention provides a method of making a compound according to the first and second aspects of the invention, said method comprising expressing one or more nucleic acid molecules according to the third aspect of the invention in a host cell and isolating the compound therefrom.

It is preferable that the two portions of the compound of the invention are produced as a fusion compound by recombinant DNA techniques, whereby a length of DNA comprises respective regions encoding the two portions of the compound of the invention either adjacent one another or separated by a region encoding a linker peptide which does not destroy the desired properties of the compound. The benefits in making the compound of the invention using recombinant DNA techniques are several fold. Firstly, it enables a high degree of precision with which the two portions of the compound can be joined together. Secondly, the construction of compounds which are "hetero-oligomeric" can be controlled by the expression of the different recombinant DNA molecules encoding each of the different type of subunit of the "hetero-oligomer" in the same host cell.

By "hetero-oligomer" we mean those compounds in which two or more different cell-specific portions are joined to either the same or to different subunits which are capable of oligomerisation. The expression, in the same host cell of two compounds, of A and B, each with different target cell specific portions but with a common second portion capable of oligomerisation will result in a mixed population of compounds. For example, if the common second portion is capable of dimerisation, three potential compounds will be produced: A₂, AB and B₂, in a ratio of 1:2:1, respectively.

The separation of the desired compound with each of the different cell specific portions, that is AB, can be achieved by two step affinity chromatography.

Application of the mixture of compounds to an affinity column specific for A will result in the binding of A₂ and AB. These compounds are eluted from this first column, and then applied to an affinity column specific for B. This will result in AB, but not A₂, being bound to the column. Finally, the desired product AB, can be eluted.

Of course, the order in which the affinity columns are used is not important.

The same principle of separating those compounds with two or more different binding sites can be applied to the purification of the desired compounds from mixtures of other hetero-oligomers.

Conceivably, the two portions of the compound may overlap wholly or partly.

The DNA is then expressed in a suitable host to produce a polypeptide comprising the compound of the invention. Thus, the DNA encoding the polypeptide constituting the compound of the invention may be used in accordance with known techniques, appropriately modified in view of the teachings contained herein, to construct an expression vector, which is then used to transform an appropriate host cell for the expression and production of the polypeptide of the invention. Such techniques include those disclosed in US Patent Nos. 4,440,859 issued 3 April 1984 to Rutter *et al,* 4,530,901 issued 23 July 1985 to Weissman, 4,582,800 issued 15 April 1986 to Crowl, 4,677,063 issued 30 June 1987 to Mark *et al,* 4,678,751 issued 7 July 1987 to Goeddel, 4,704,362 issued 3 November 1987 to Itakura *et al,* 4,710,463 issued 1 December 1987 to Murray, 4,757,006 issued 12 July 1988 to Toole, Jr. *et al*, 4,766,075 issued 23 August 1988 to Goeddel *et al* and 4,810,648 issued 7 March 1989 to Stalker.

The DNA encoding the polypeptide constituting the compound of the invention may be joined to a wide variety of other DNA sequences for introduction into an appropriate host. The companion DNA will depend upon the nature of the host, the manner of the introduction of the DNA into the host, and whether episomal maintenance or integration is desired. Generally, the DNA is inserted into an expression vector, such as a plasmid, in proper orientation and correct reading frame for expression. If necessary, the DNA may be linked to the appropriate transcriptional and transiational regulatory control nucleotide sequences recognised by the desired host, although such controls are generally available in the expression vector. The vector is then introduced into the host through standard techniques. Generally, not all of the hosts will be transformed by the vector. Therefore, it will be necessary to select for transformed host cells. One selection technique involves incorporating into the expression vector a DNA sequence, with any necessary control elements, that codes for a selectable trait in the transformed cell, such as antibiotic resistance. Alternatively, the gene for such selectable trait can be on another vector, which is used to co-transform the desired host cell.

Host cells that have been transformed by the recombinant DNA of the invention are then cultured for a sufficient time and under appropriate conditions known to those skilled in the art in view of the teachings disclosed herein to permit the expression of the polypeptide, which can then be recovered.

Many expression systems are known, including bacteria (for example *E. coli* and *Bacillus subtilis*), yeasts (for example *Saccharomyces cerevisiae* and *Pichia pastoris*), filamentous fungi (for example *Aspergillus*), plant cells, animal cells (for example COS-1, COS-7, CHO, MH 3T3, NS0 and BHK cells) and insect cells (for example Drosophila, SF9 cells).

Those vectors that include a replicon such as a procaryotic replicon can also include an appropriate promoter such as a procaryotic promoter capable of directing the expression (transcription and translation) of the genes in a bacterial host cell, such as *E. coli,* transformed therewith.

A promoter is an expression control element formed by a DNA sequence that permits binding of RNA polymerase and transcription to occur. Promoter sequences compatible with exemplary bacterial hosts are typically provided in plasmid vectors containing convenient restriction sites for insertion of a DNA segment of the present invention.

Typical procaryotic vector plasmids are pUC18, pUC19, pBR322 and pBR329 (available from Biorad Laboratories, Richmond, CA, USA), p*Trc*99A and pKK223-3 (available from Pharmacia Piscataway, NJ, USA) and the pET system (T7 promoter, Novagen Ltd).

A typical mammalian cell vector plasmid is pSVL available from Pharmacia, Piscataway, NJ, USA. This vector uses the SV40 late promoter to drive expression of cloned genes, the highest level of expression being found in T antigen-producing cells, such as COS-1 cells.

An example of an inducible mammalian expression vector is pMSG, also available from Pharmacia. This vector uses the glucocorticoid-inducible promoter of the mouse mammary tumour virus long terminal repeat to drive expression of the cloned gene.

Useful yeast plasmid vectors are pRS403-406 and pRS413-416 and are generally available from Stratagene Cloning Systems, La Jolla, CA 92037, USA. Plasmids pRS403, pRS404, pRS405 and pRS406 are Yeast Integrating plasmids (YIps) and incorporate the yeast selectable markers *his3, trp1, leu2* and *ura3*. Plasmids pRS413-416 are Yeast Centromere plasmids (YCps).

Further useful vectors for transformation of yeast cells, such as *Pichia,* include the 2µ plasmid pYX243 (available from R and D Systems Limited) and the integrating vector pPICZ series (available from Invitrogen).

A variety of methods have been developed to operatively link DNA to vectors via complementary cohesive termini. For instance, complementary homopolymer tracts can be added to the DNA segment to be inserted to the vector DNA. The vector and DNA segment are then joined by hydrogen bonding between the complementary homopolymeric tails to form recombinant DNA molecules.

Synthetic linkers containing one or more restriction sites provide an alternative method of joining the DNA segment to vectors. The DNA segment, generated by endonuclease restriction digestion as described earlier, is treated with bacteriophage T4 DNA polymerase or *E. coli* DNA polymerase I, enzymes that remove protruding, 3'-single-stranded termini with their 3'-5'-exonucleolytic activities, and fill in recessed 3'-ends with their polymerizing activities.

The combination of these activities therefore generates blunt-ended DNA segments. The blunt-ended segments are then incubated with a large molar excess of linker molecules in the presence of an enzyme that is able to catalyze the ligation of blunt-ended DNA molecules, such as bacteriophage T4 DNA ligase. Thus, the products of the reaction are DNA segments carrying polymeric linker sequences at their ends. These DNA segments are then cleaved with the appropriate restriction enzyme and ligated to an expression vector that has been cleaved with an enzyme that produces termini compatible with those of the DNA segment.

Synthetic linkers containing a variety of restriction endonuclease sites are commercially available from a number of sources including International Biotechnologies Inc, New Haven, CN, USA.

A desirable way to modify the DNA encoding the polypeptide of the invention is to use the polymerase chain reaction as disclosed by Sailor *et al* (1988) *Science* **239,** 487-491.

In this method the DNA to be enzymatically amplified is flanked by two specific oligonucleotide primers which themselves become incorporated into the amplified DNA. The said specific primers may contain restriction endonuclease recognition sites which can be used for cloning into expression vectors using methods known in the art.

Exemplary genera of yeast contemplated to be useful in the practice of the present invention are *Pichia, Saccharomyces, Kluyveromyces, Candida, Torulopsis, Hansenula, Schizosaccharomyces, Citeromyces, Pachysolen, Debaromyces, Metschunikowia, Rhodosporidium, Leucosporidium, Botryoascus, Sporidiobolus, Endomycopsis*, and the like. Preferred genera are those selected from the group consisting of *Pichia, Saccharomyces, Kluyveromyces, Yarrowia* and *Hansenula*. Examples of *Saccharomyces* are *Saccharomyces cerevisiae, Saccharomyces italicus* and *Saccharomyces rouxii*. Examples of *Kluyveromyces* are *Kluyveromyces fragilis* and *Kluyveromyces lactis.* Examples of *Hansenula* are *Hansenula polymorpha, Hansenula anomala* and *Hansenula capsulata. Yarrowia lipolytica* is an example of a suitable *Yarrowia* species.

Methods for the transformation of *S. cerevisiae* are taught generally in EP 251 744, EP 258 067 and WO 90/01063, all of which are incorporated herein by reference.

Suitable promoters for *S. cerevisiae* include those associated with the *PGK1* gene, *GAL1* or *GAL10* genes, *CYC1, PH05, TRP1, ADH1, ADH2,* the genes for glyceraldehyde-3-phosphate dehydrogenase, hexokinase, pyruvate decarboxylase, phosphofructokinase, triose phosphate isomerase, phosphoglucose isomerase, glucokinase, α-mating factor pheromone, a-mating factor pheromone, the *PRB1* promoter, the *GUT2* promoter, and hybrid promoters involving hybrids of parts of 5' regulatory regions with parts of 5' regulatory regions of other promoters or with upstream activation sites (eg the promoter of EP-A-258 067).

The transcription termination signal is preferably the 3' flanking sequence of a eukaryotic gene which contains proper signals for transcription termination and polyadenylation. Suitable 3' flanking sequences may, for example, be those of the gene naturally linked to the expression control sequence used, i.e. may correspond to the promoter. Alternatively, they may be different in which case the termination signal of the *S. cerevisiae AHD1* gene is preferred.

The present invention also relates to a host cell transformed with a polynucleotide vector construct of the present invention. The host cell can be either procaryotic or eukaryotic. Bacterial cells are preferred procaryotic host cells and typically are a strain of *E. coli* such as, for example, the *E. coli* strains DH5 available from Bethesda Research Laboratories Inc., Bethesda, MD, USA, and RR1 available from the American Type Culture Collection (ATCC) of Rockville, MD, USA (No ATCC 31343). Preferred eukaryotic host cells include yeast and mammalian cells, preferably vertebrate cells such as those from a mouse, rat, monkey or human fibroblastic cell line. Preferred eukaryotic host cells include Chinese hamster ovary (CHO) cells available from the ATCC as CCL61, NIH Swiss mouse embryo cells NIH/3T3 available from the ATCC as CRL 1658 and monkey kidney-derived COS-1 cells available from the ATCC as CRL 1650 or WSØ cells.

Transformation of appropriate cell hosts with a DNA construct of the present invention is accomplished by well known methods that typically depend on the type of vector used. With regard to transformation of procaryotic host cells, see, *for* example, Cohen *et al, Proc. Natl. Acad. Sci. USA,* **69:** 2110 (1972); and Sambrook *et al, Molecular Cloning, A Laboratory Manual*, Cold Spring Harbor Laboratory, Cold Spring Harbor, NY (1989). Transformation of yeast cells is described in Sherman *et al, Methods In Yeast Genetics, A Laboratory Manual,* Cold Spring Harbor, NY (1986). The method of Beggs, *Nature*, **275:** 104-109 (1978) is also useful. With regard to vertebrate cells, reagents useful in transfecting such cells, for example calcium phosphate and DEAF-dextran or liposome formulations, are available from Stratagene Cloning Systems, or Life Technologies Inc, Gailliersburg, MD 20877, USA.

Successfully transformed cells, *i.e*. cells that contain a DNA construct of the present invention, can be identified by well known techniques. For example, cells resulting from the introduction of an expression construct of the present invention can be grown to produce the polypeptide of the invention. Cells can be harvested and lysed and their DNA content examined for the presence of the DNA using a method such as that described by Southern, *J. Mol. Biol*., **98:** 503 (1975) or Berent *et al, Biotech*., **3:** 208 (1985). Alternatively, the presence of the protein in the supernatant can be detected using antibodies as described below.

In addition to directly assaying for the presence of recombinant DNA, successful transformation can be confirmed by well known immunological methods when the recombinant DNA is capable of directing the expression of the protein. For example, cells successfully transformed with an expression vector produce proteins displaying appropriate antigenicity. Samples of cells suspected of being transformed are harvested and assayed for the protein using suitable antibodies.

Thus, in addition to the transformed host cells themselves, the present invention also contemplates a culture of those cells, preferably a monoclonal (clonally homogeneous) culture, or a culture derived from a monoclonal culture, in a nutrient medium. Preferably, the culture also contains the protein.

Nutrient media useful for culturing transformed host cells are well known in the art and can be obtained from several commercial sources.

A fifth aspect of the invention provides a vector for expression of a compound according to the first or second aspect of the invention, or a target cell specific portion, mediator portion or cytotoxic portion thereof, said vector comprising a nucleic acid molecule according to the fourth aspect of the invention.

A sixth aspect of the invention provides a host cell comprising a nucleic acid molecule according to the fourth aspect of the invention or a vector according to the fifth aspect of the invention.

Preferably, the host cell is a bacterial cell or a yeast cell.

A seventh aspect of the invention provides a pharmaceutical composition comprising a compound according to the first or second aspect of the invention and a pharmaceutically acceptable carrier or excipient.

The compounds and compositions of the invention are administered in any suitable way, usually parenterally, for example intravenously, intraperitoneally or, preferably (for bladder cancer), intravesically (*i.e*. into the bladder), in standard sterile, non-pyrogenic formulations of diluents and carriers, for example isotonic saline (when administered intravenously).

A eighth aspect of the invention provides a compound according to the first or second aspect of the invention or a composition according to the seventh aspect of the invention for use in medicine.

The compounds and compositions of the invention may be used to treat a patient with any disease involving a dysfunction of a population of cells, said compounds and compositions selectively targeting and destroying said population of cells within a patient. For example, said compounds and compositions may be used in the treatment of cancer, *e.g.* cancer of the breast, ovaries, lung, stomach, intestines, blood *etc.* Thus, anti-tumour cell antigen antibodies can be used to deliver a cytotoxic portion with caspase apoptosis-inducing activity to a tumour cell. Antibodies that are internalised upon contact with the target antigen are used, such that the cytotoxic portion enters the cytosol of the tumour cell, where it can trigger off an apoptotic cascade.

In principle, the compounds and compositions of the invention may be used to treat any mammal, including pets such as dogs and cats and agriculturally important animals such as cows, horses, sheep and pigs.

Preferably, the patient is human.

A further aspect of the invention provides the use of a compound according to the first or second aspect of the invention in the preparation of a medicament for treating a disease associated with the dysfunction of a population of cells.

Preferably, the medicament is for treating cancer.

Yet another aspect of the invention provides a method-of treating a patient having target cells to be destroyed, said method comprising administering a therapeutically effective amount of a compound or composition of the invention to said patient.

Preferably the patient has cancer.

The invention will now be described in detail with reference to the following figures and examples:
Figure 1 shows schematic examples of the various types of compounds of the invention.
Figure 2 shows a schematic diagram showing the construction of the gene expressing the HMFG 1 Fab-Caspase fusion protein. Steps A to G are described in the text below. Note that the final construct pET20HMFGLSCasp3 contains the VL-CL expression cassette in the Bpu1102 I site. An alternative construct, lacking the pel B secretion signal for cytosolic expression is constructed the same way, using pET21b as the starting vector, and the final clone is called pET21HMFGLSCasp3.
Figure 3 shows the continuous nucleotide sequence of the construct which when expressed in *E. coli*, will assemble to form a HMFG1 Fab/Caspase-3 fusion protein. The vector backbone is pET20b. Sequence reads as follows:
   Pel B secretion signal (underlined) 1-66; VH domain (italics) 67-420; CH1 domain 421-714; Linker peptide (underlined) 715-759; rearranged caspase-3/CPP-32 (italics) 760-1277; Linker and Polyhistidine Tag sequence (underlined) 1278-1320. Additional T7 promoter, and ribosome binding site 1321-1461; Pel B secretion signal (underlined) 1462-1527; VL domain 1462-1869; CL domain 1870-2193.

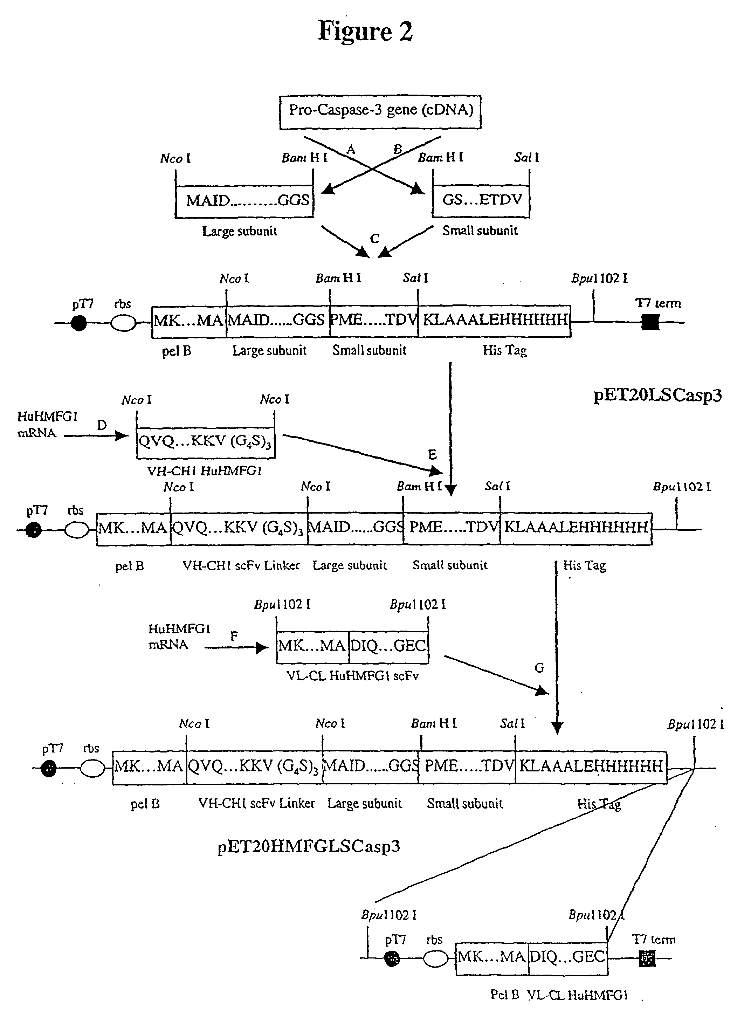

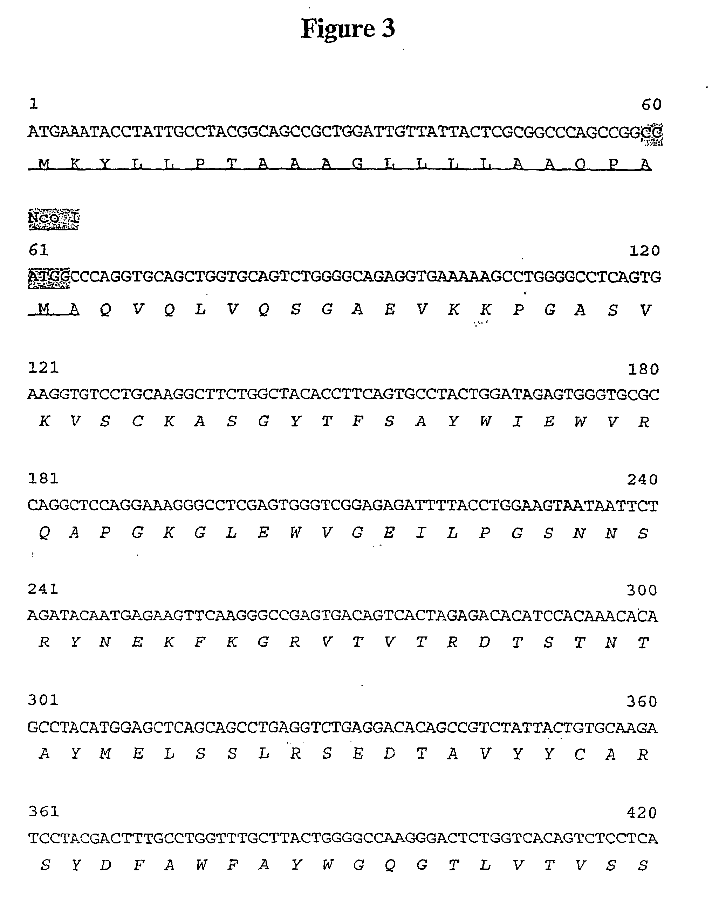

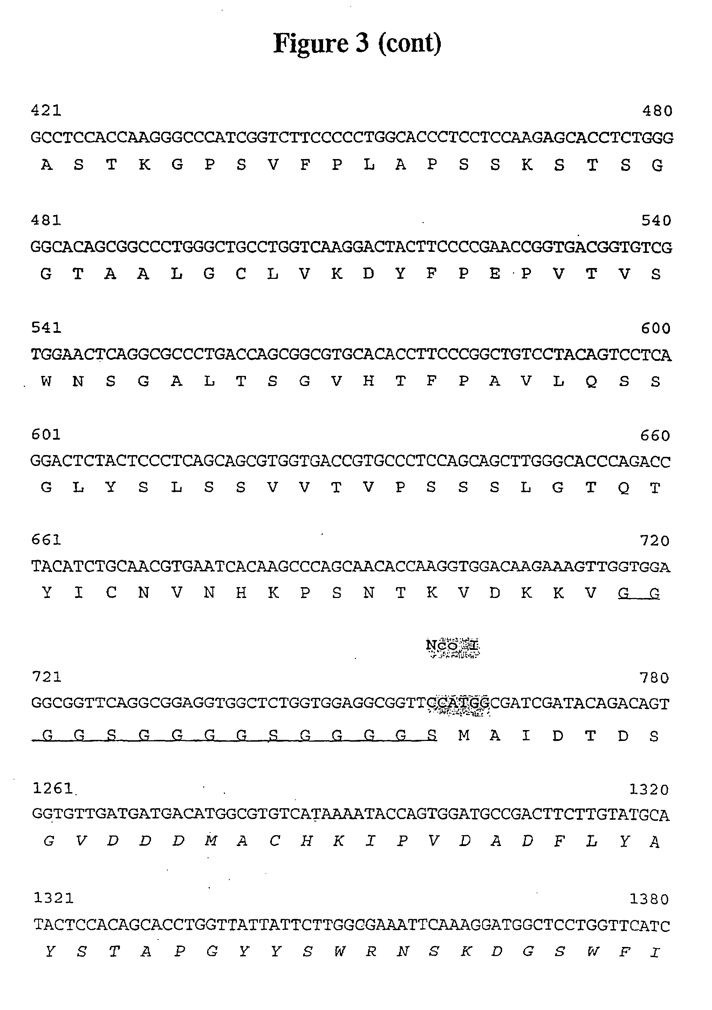

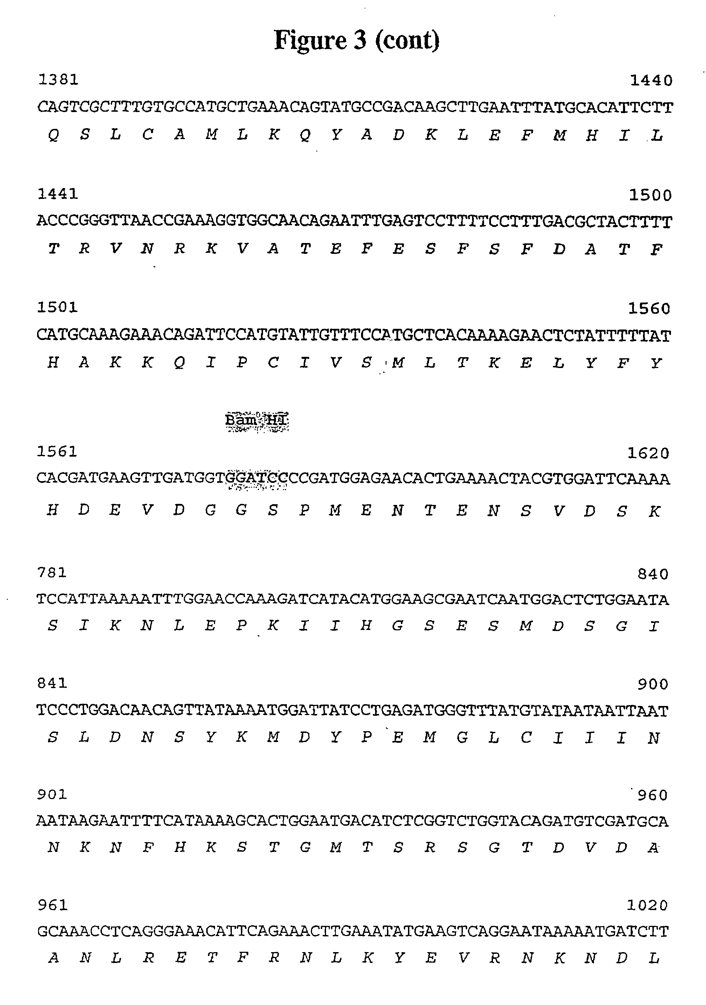

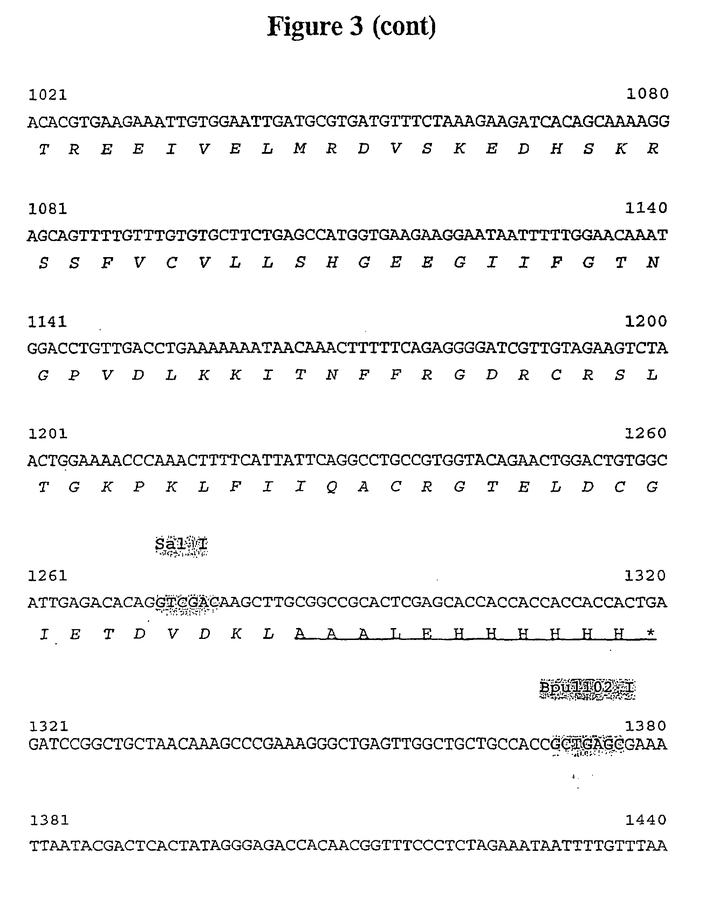

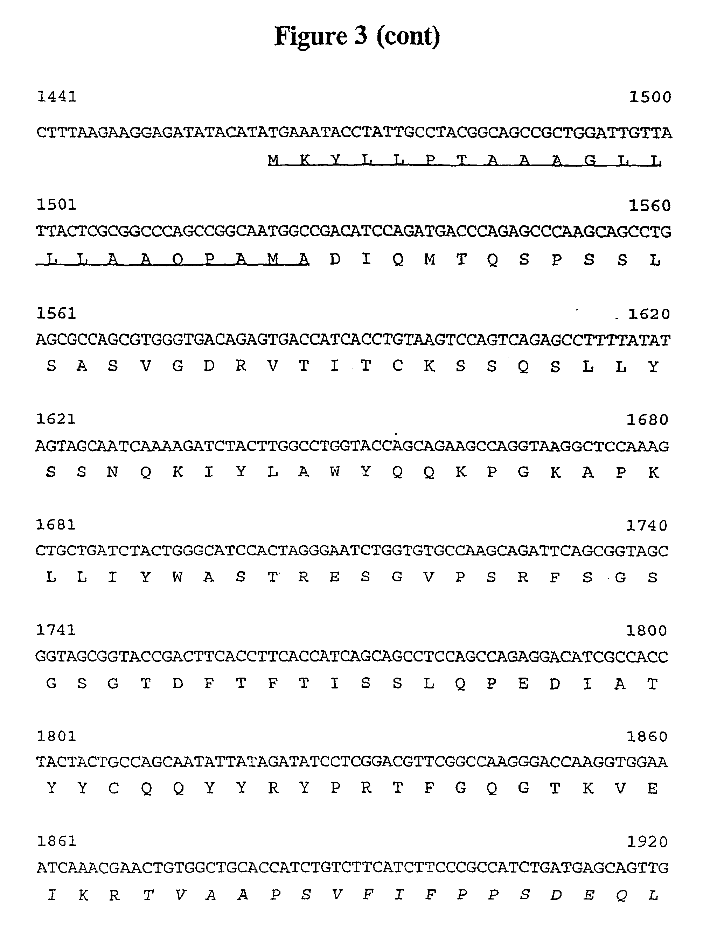

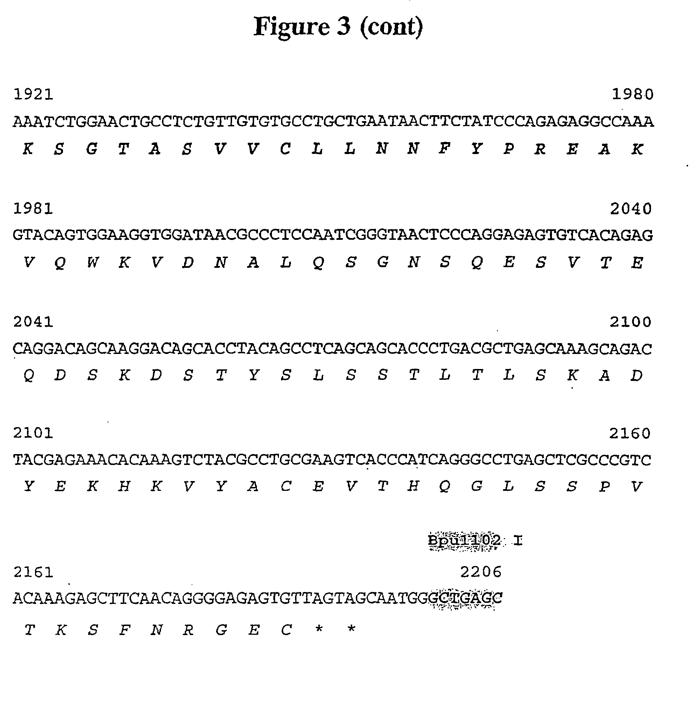

The restriction sites referred to in Figure 2 are presented in bold in Figure 3.

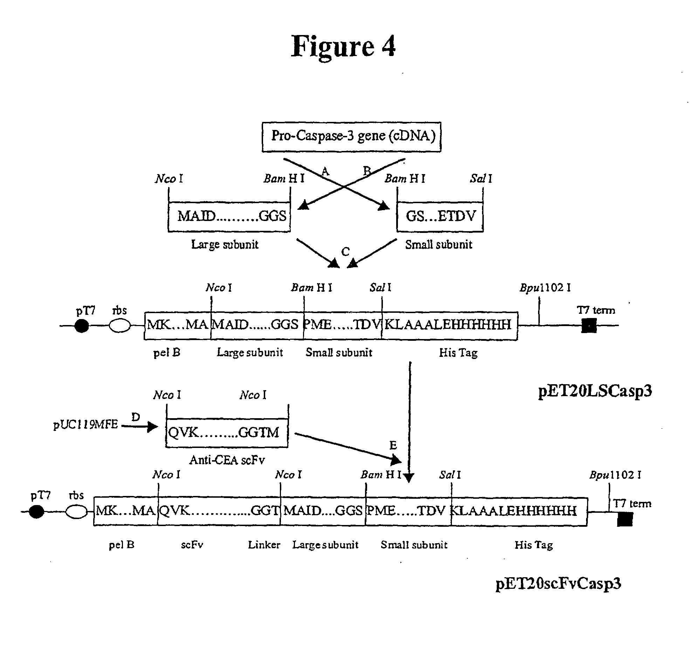

Figure 4 shows a schematic diagram showing the construction of the vector (pET20scFvCasp3) expressing the scFv rearranged-Caspase 3 fusion protein. Steps A to E are described in the text. An alternative construct, lacking the *pel* B secretion signal for cytosolic expression is constructed the same way, using pET21b as the starting vector, and the final clone is called pET21scFvSCasp3. Flanking amino acid sequences are shown.

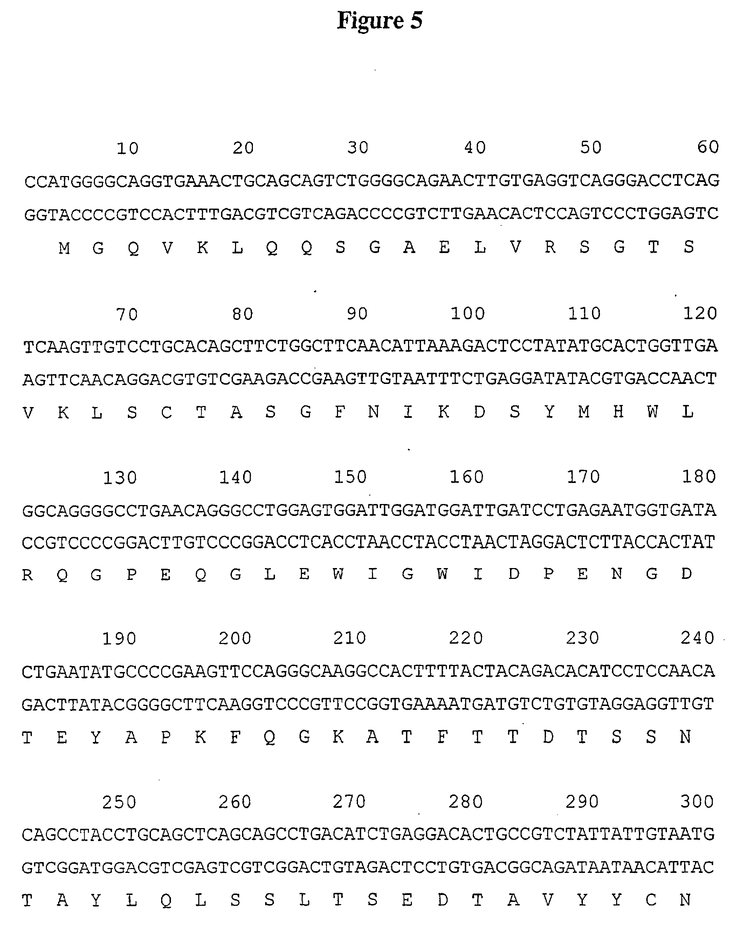

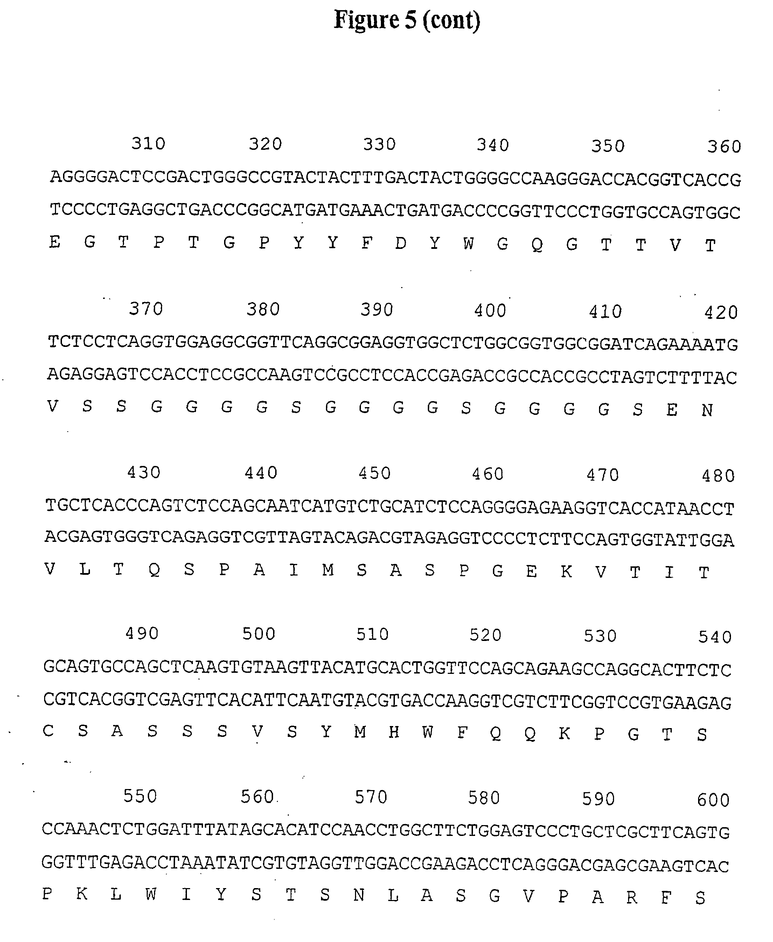

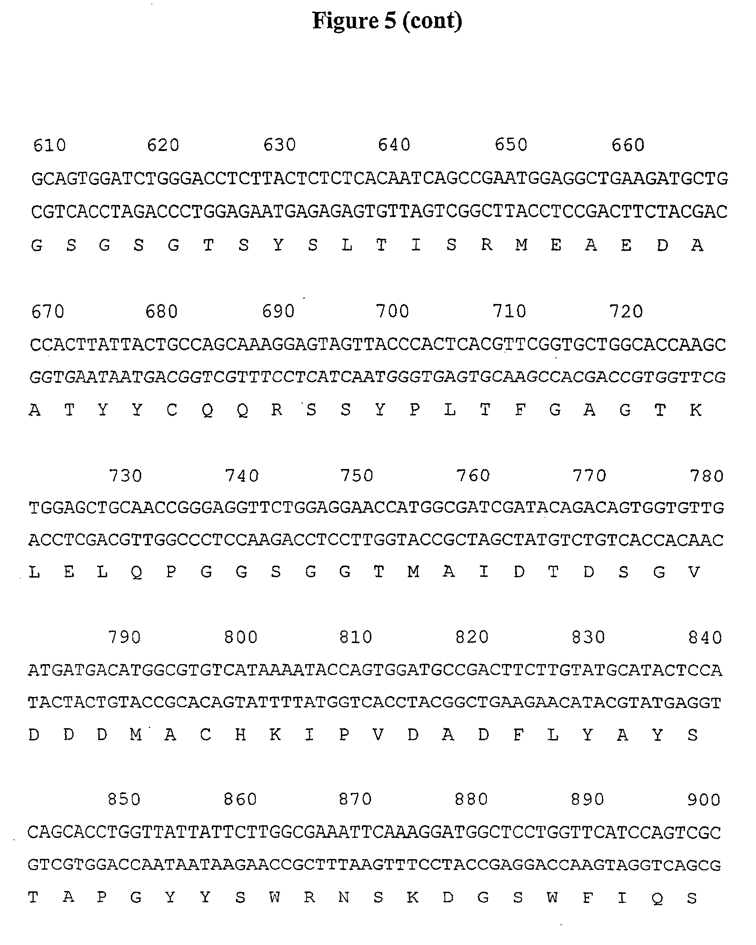

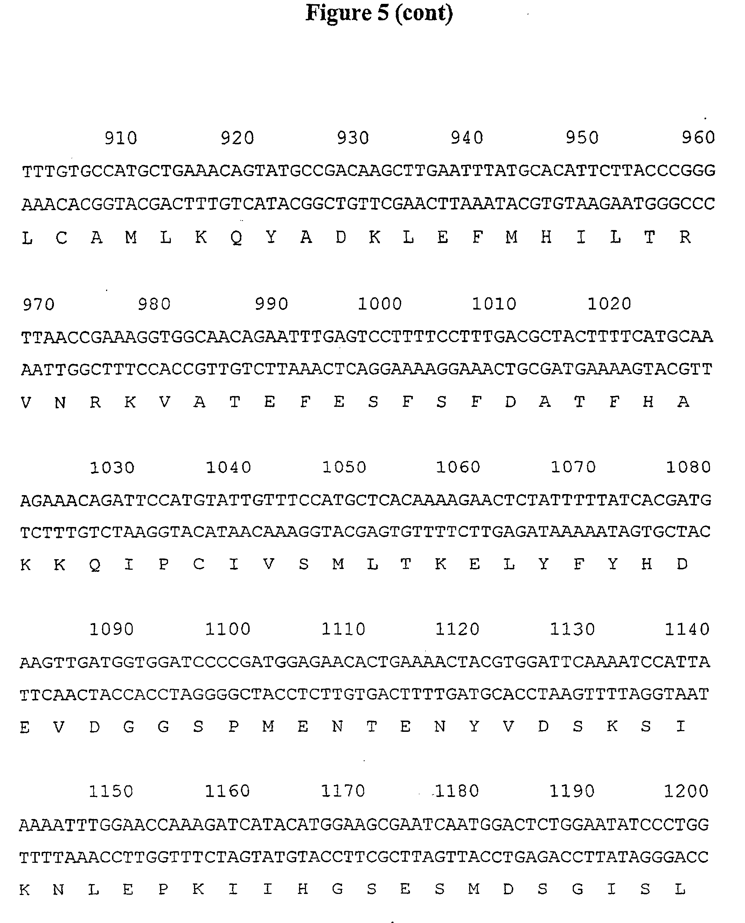

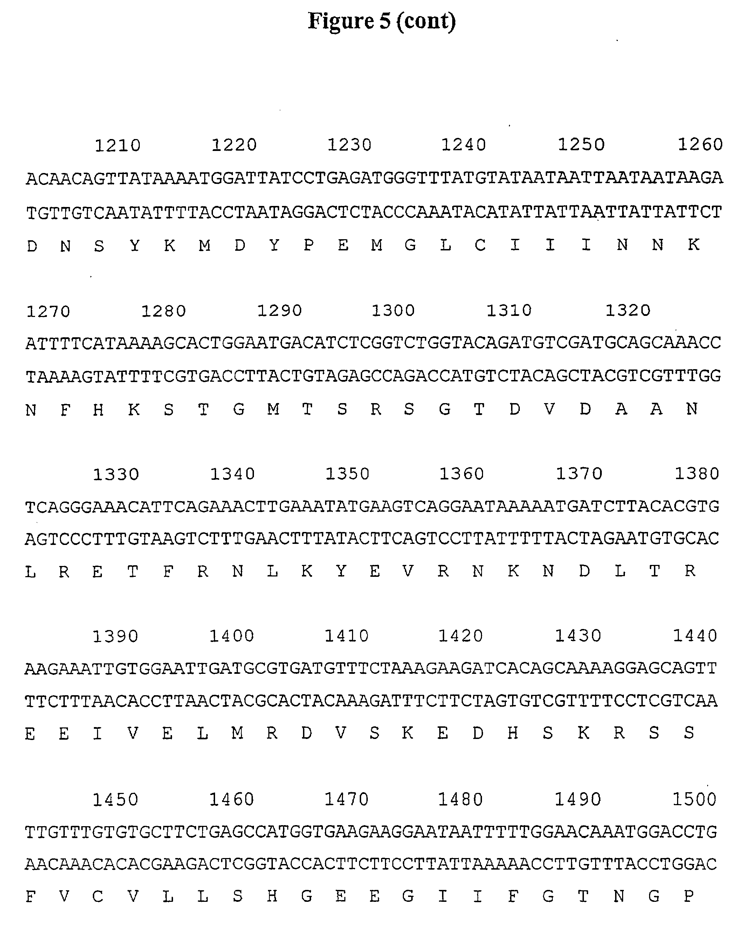

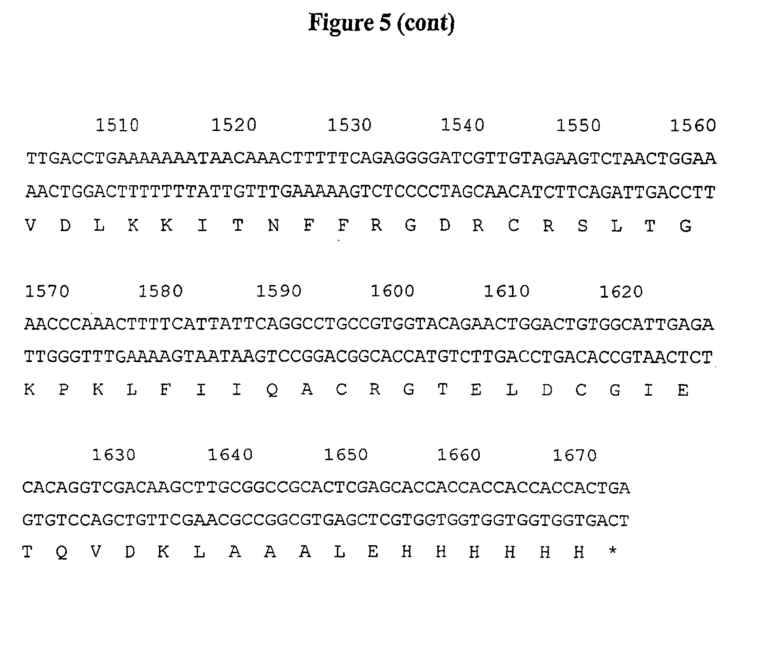

Figure 5 shows the continuous nucleotide and amino acid sequence of the fusion gene which when expressed in *E. coli,* will produce the scFv-rearranged caspase 3 fusion protein. The vector backbone is pET20b. Sequence reads as follows: end of *pel* B from pET20b vector (1-8), scFv (9-731), linker (732-752), rearranged caspase (753-1652), His-tag (1653-1673).

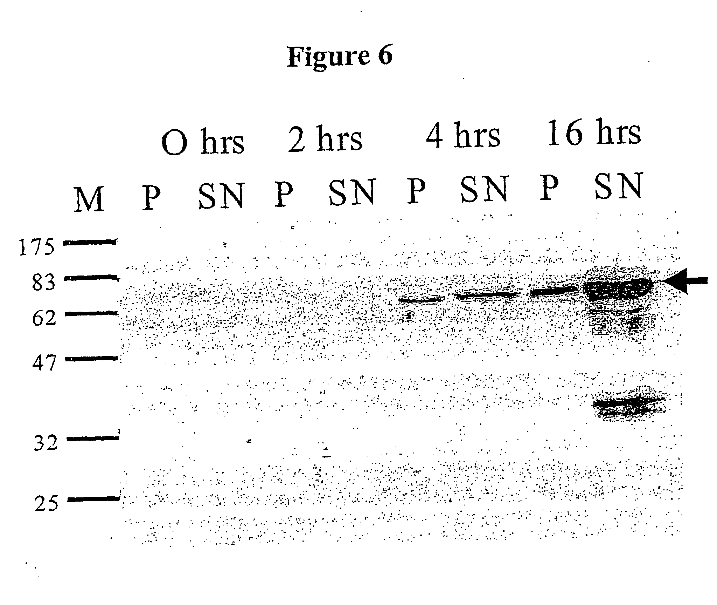

Figure 6 shows expression in *E. coli* BL21(DE3)pLysS of the scFv-rearranged caspase 3 fusion protein from vector pET20scFvcasp3. P=cell pellet, SN=bacterial supernatant, M=molecular weight markers. Time course is 0 to 16 hrs. Arrow indicates position of scFv-rearranged caspase 3 protein.

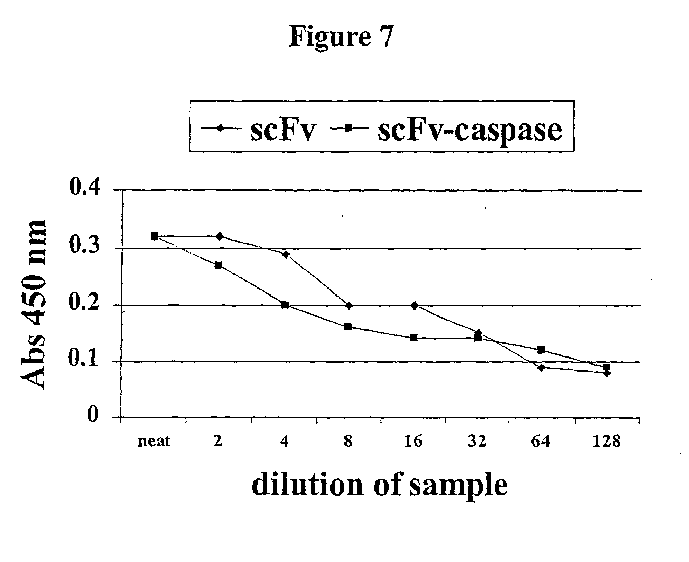

Figure 7 shows ELISA on pure CEA of the scFv and scFv- rearranged caspase 3 fusion protein, developed with anti-HIS antibody (Qiagen). The starting concentration of the proteins were 1 µg/ml in PBS.

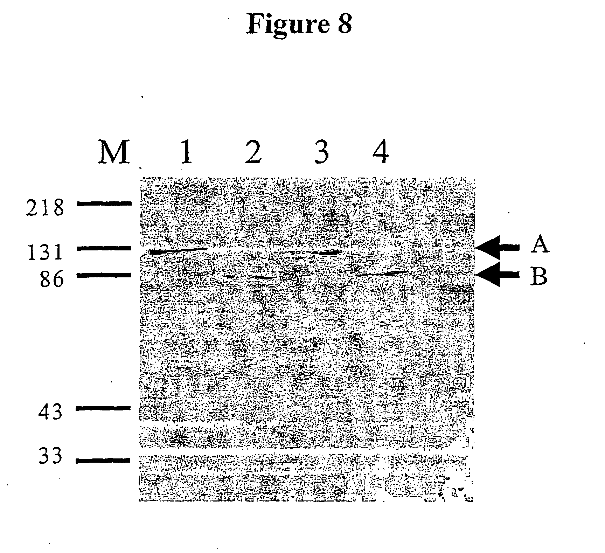

Figure 8 shows PARP cleavage assay of the scFv and scFv- rearranged caspase 3 fusion protein a cell lysate from SKOV3 cells, which contains full length PARP is incubated with the tested proteins for 1 hr at 37 °C. The full length (arrow A, 130 KDa) and cleaved PARP (arrow B, 85 KDa) are detected by western blot. Lanes are as follows: (1) Cell lysate, untreated, (2) Cell lysate treated with scFv-rearranged caspase fusion protein, (3) Cell lysate treated with scFv only, (4) Cell lysate treated with pure caspase. Detecting antibody as anti-PARP (Santa Cruz biochemicals Ltd).

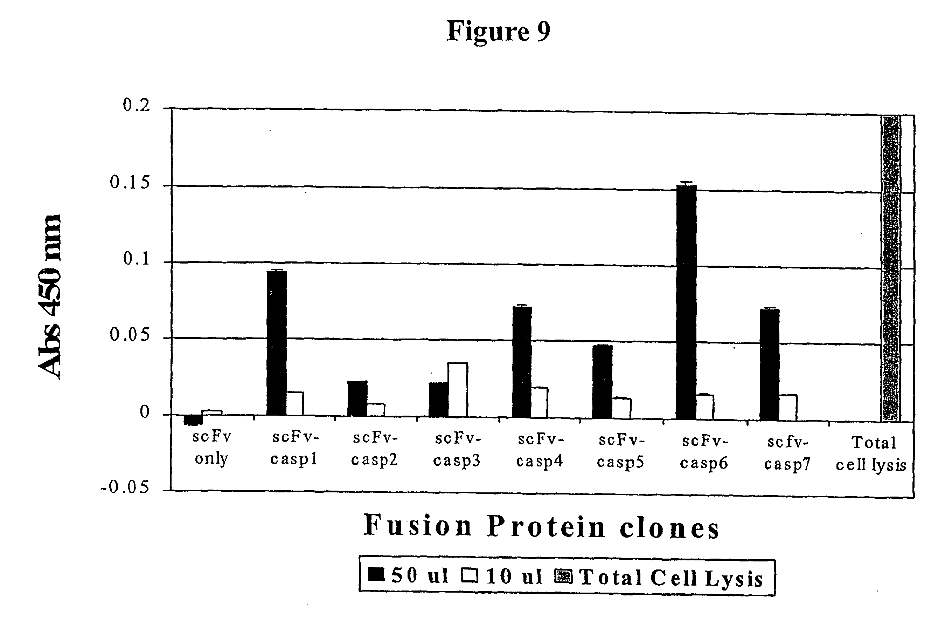

Figure 9 shows cytotoxicity of 7 selected scFv-caspase 3 fusion proteins and the anti-CEA scFv alone. Fifty and ten microlitres of concentrated bacterial supernatant were added to LS174T for 48 hr. Background values for untreated cells were subtracted to give actual cell lysis values as measured by the Cytotox-96 kit.

### EXAMPLES

### (A) Expression of constitutively active caspases

### Construction of a nucleic acid molecule encoding a constitutively active caspase

PCR amplification is used to produce a DNA construct encoding a constitutively active caspase-3, wherein the order of the small and large subunits is reversed, as described in Srinivasula *et al*. (1998) *J. Biol. Chem*. **273**:10107-10111.

### Generation of cDNAs expressing precursors of constitutively active Caspase-3 and Caspase-6

Complementary DNAs (cDNAs) encoding precursors of constitutively active caspase-3 and caspase-6 are generated by PCR. The large and small subunits of caspase-3 were amplified with the following primers, using the caspase-3 DNA as a template:
Caspase-3 large subunit forward primer: ATGGAGAACACTGAAAACTCAG
Caspase-3 large reverse primer: GTCATCATCAACACCTCAGTCT
Caspase-3 small subunit forward primer: GGATCCATGATTGAGACAGACAGTGG
Caspase-3 small reverse primer:

ATCAACTTCATCGTGATAAAAATAGAGTTC

The caspase-3 PCR products are cloned separately into the *Sma*I site of pBluescript KS⁺. The small subunit is then excised from the KS⁺-vector with *Bam*HI and inserted into the *Bam*HI site of the second KS⁺-vector, which contains the large subunit. This places the small subunit in-frame with the large subunit.

The precursor of constitutively active caspase-6 is amplified and cloned in the KS⁺-vector in a similar way. The following PCR primers are used with caspase-6-His₆ as a template:
Caspase-6 large subunit forward primer: ATGAGCTCGGCCTCGGGG
Caspase-6 large subunit reverse primer: TTAATCTACTACATCCAAAGG
Caspase-6 small subunit forward primer: GGATCCATGGTAGAAATAGATGCAGCCTCCGTTTAC
Caspase-6 small subunit reverse primer:

ATCAATTTCAACGTGGTGGTGGTGGTGGTGC

To express the constitutively active caspases in bacteria (BL21-DE3), their cDNAs are excised with *Bam*H1/*Xho*I and subcloned into the bacterial expression vector pET28a in-frame with the His₆-T7-tag of this vector.

### Expression of constitutively active caspases in mammalian cells and assay of apoptosis

To express the constitutively active caspase in mammalian cells and assay their apoptotic activity, they are amplified with the T7-tag primer and the large subunit reverse primers using the pET28a constructs as templates, and subcloned into the mammalian double expression vector pRSC-LacZ (see MacFarlane *et al.,* 1997, *J. Biol. Chem.* **272:**25417-25420). This vector allows the expression of *lacZ* under the Rous sarcoma virus promoter and the *test* DNA under the cytomegalovirus promoter. It also allows the *in vitro* transcription of the test cDNA from the T7-promoter.

To assay for apoptosis, MCF-7 or 293 cells are transfected with the pRSC-LacZ constructs in the presence or absence of different apoptosis inhibitors. The cells are stained with β-galactosidase 30 h after transfection and examined for morphological signs of apoptosis. The percentage of round blue apoptotic cells as compered to total blue cells gives a marker of apoptosis.

### In vitro translation of caspases

Caspases are *in vitro* translated in the presence of [³⁵S]methionine in rabbit reticulocytes lysate with a T7-RNA polymerase-coupled TNT kit (Promega), using the pRSC-LacZ or pET28a constructs as templates according to the manufacturer's recommendations.

### (B) Construction and production of a HMFG-1 Fab portion/Caspase 3 recombinant fusion protein

1. (a) The Caspase-3 gene is used as a template to amplify (by PCR) the small subunit sub-gene using primers 'CASP3SMFW and CASP3SMBK (see Table 4), which contains flanking *Bam* H I and *Sal* I restriction sites (Fig. 2, reaction A).
(b) The Caspase-3 gene is used as a template to amplify (by PCR) the large subunit sub-gene using primers CASP3LGFW and CASP3LGBK (see Table 4), which contains flanking *Nco* I and *Bam* HI restriction sites (Fig. 2, reaction B). 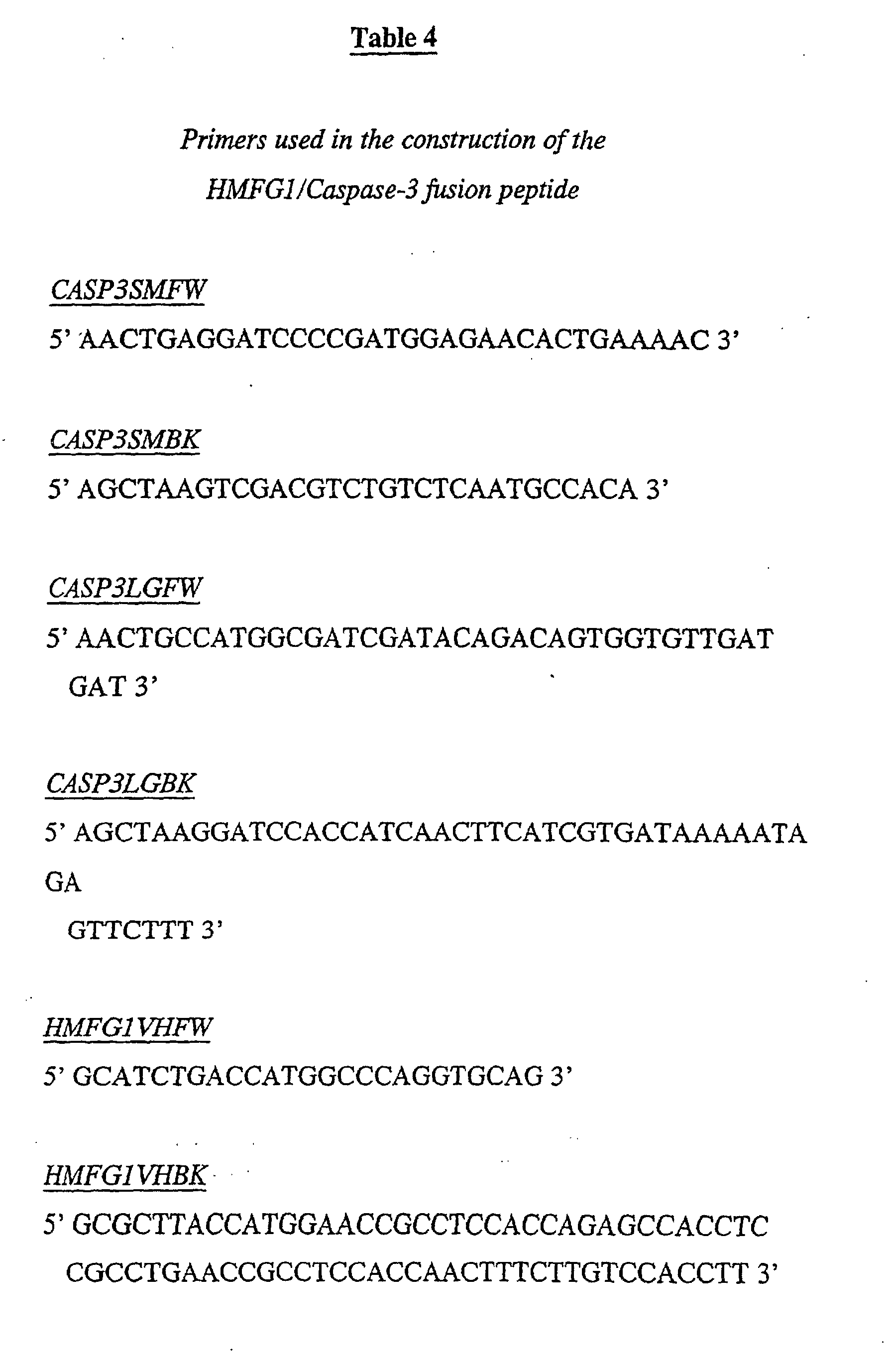 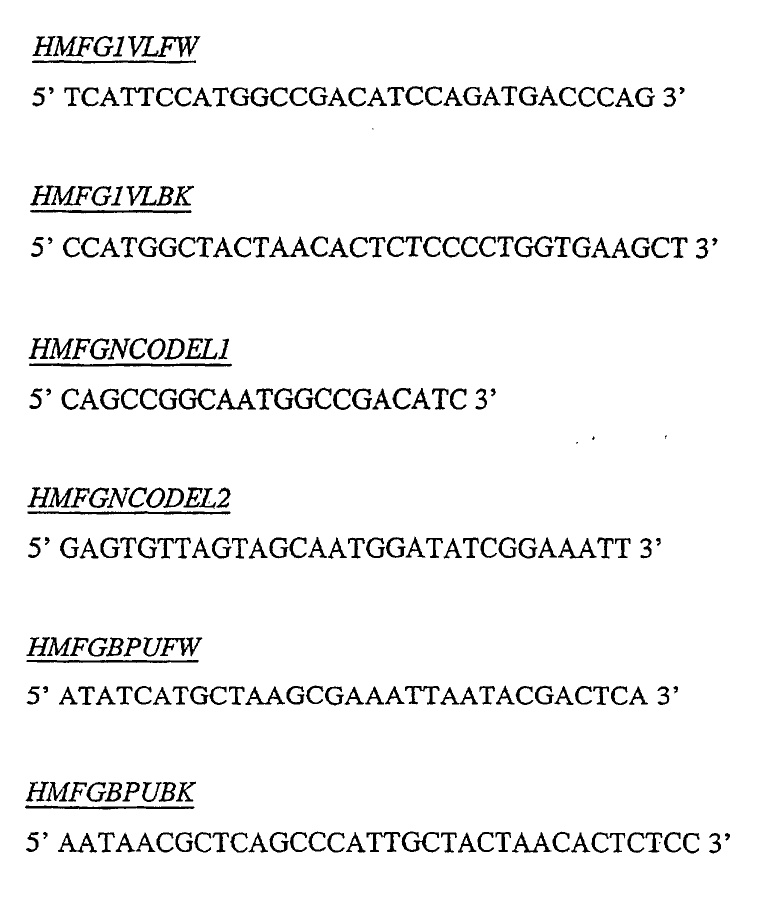
2. The large subunit subgene PCR product is digested with *Nco* I/*Bam HI* restriction enzymes and ligated into the *Nco* I/*Bam* HI sites of pET20b expression vector (Novagen; see Fig. 2, reaction C). The resulting plasmid is called pET20LCasp3. The small subunit subgene PCR product is digested and ligated into the *Bam* HI/*Sal* I sites of the pET20LCasp3 plasmid to form the plasmid pET20LSCasp3 (Fig. 2, reaction C). This construct can be used to express the active caspase-3 alone, in *E. coli,* to test for activity and characterise the recombinant enzyme.
3. HMFG-1 mRNA is produced from the cell culture of the HMFG-1 hybridoma (ICRF), using the RNAse easy kit (Qiagen). HMFG-1 cDNA is made by reverse transcription of the mRNA using oligo-dT primers and a RT-PCR kit (Qiagen). The VH-CH1 subgene of the HMFG-1 immunoglobulin is amplified by PCR from the cDNA using the primers HMFG1VHFW and HMFG1VHBK (Fig. 2, reaction D). The PCR product is digested with Nco I and ligated into the Nco I site of pET20LSCasp3 (Fig. 2, reaction E). The resulting plasmid is pET20HVHCasp3.
4. The HMFG-1 cDNA from (4) is used to PCR amplify the VL-CL subgene using the primers HMFG1VLFW and HMFG1VLBK, to produce a cassette with flanking *Nco* I sites. The PCR product is digested with *Nco* I and ligated into the *Nco* I site of pET20b. The two Nco I sites are removed by oligodirected mutagenesis, following the Amersham-Pharmacia mutagenesis kit (version 2) protocol, and primers HMFGNCODEL1 and HMFGNCODEL2. The resulting plasmid is used as the template to amplify the expression cassette to clone into the pET20VHCasp3, using primers HMFGBPUFW and HMFGBPUBK, which puts flanking Bpu1102 I sites (Fig. 2, reaction F. The PCR product is digested with Bpu1102 I and ligated into the Bpu1102 site of pET20HVHCasp3 (Fig. 2 reaction G). The resulting plasmid is a bicistronic vector expressing an HMFG-1 Fab-Caspase 3 fusion protein, called pET20HMFGLSCasp3.
5. Alternatively, the fusion protein can be constructed following the identical scheme (steps 1 to 5), but using the vector pET21d, for cytosolic expression. The *E. coli* strain used is BL21trxb (Novagen).
   *E. coli* strain BL21trxb used in this alternative method does not contain the T7 RNA polymerase gene (*i*.*e*. it is not a DE3 derivative). Hence, expression from pET-type vectors is induced by growing the cells in 2TY media with 100 µg/ml ampicillin, up to an optical density of 0.8 (at 37°C). Next, M13-T7pol virus (Invitrogen) is added (at a multiplicity of infection of 5) and the culture is allowed to grow for 1 hour. IPTG is then added to a final concentration of 1 mM followed by further ampicillin (to 100 µg/ml, assuming that all the initial ampicillin has been degraded). Induction is continued for another 3 to 5 hours.
6. The plasmid pET20HMPGLSCasp3 is transformed into B121(λDE3)pLysE (Novagen), using the calcium chloride chemical method (Sambrook *et al*., 1989, *Molecular Cloning: A Laboratory Manual* (2nd ed.) Cold Spring Harbor Laboratory, Cold Spring Harbor, New York). Transformants are plated out on 2TYagar plates (Sambrook, *supra)* containing 100µg/ml ampicillin and 35 µg/ml chloramphenicol and grown overnight at 37°C. Colonies are picked and re-straked on a fresh plate.
7. A fresh single colony is picked and grown in 5 ml 2TY media with 100µg/ml ampicillin and 35µg/ml chloramphenicol for 16 h at 37°C. This 5 ml starter culture is used to innoculate 1 litre of the same media and grown (at 30°C) until the culture density, as measured by absorbance at 600 nm, is between 0.8 and 1.0. To the culture, 10 ml of 100 mM IPTG (filter sterilised, Sigma) is added and the expression of the fusion protein is induced. The culture continues to grow under the same conditions for 3-5 h.
8. Cells are harvested by centrifugation at 10,000 g, 4 °C. The supernatant is retained, concentrated by ultrafiltration in an Amicon cell with a 30 kDa cutoff membrane, to a final volume of 10 ml. The whole cells are resuspended in 50 ml of B-PER lysis reagent (Pierce) and incubated for 30 min to lyse the cells. The lysed cells are centrifuged at 25,000g, 4°C. The supernatant from both samples are dialysed against phosphate buffered saline containing 2 mM MgCl₂ and 1 M NaCl. The dialysis involves 5-6 changes of 5 litres, for 3-4 h each at 4°C.
9. The HuHMFG-1 Fab-caspase fusion protein is purified from the cell extract or culture supernatant by metal affinity chromatography on a copper (II) chelating sepharose column (Amersham-Pharmacia Biotech). The samples are pooled and loaded in PBS/1 M NaCl (column buffer) onto about 5 ml of resin. The column is washed with 100 ml of column buffer. Next, a 0-200 mM imidazole gradient is applied over a total volume of 200 ml. The fusion protein should elute between 80-150 mM imidazole.
10. Fractions from the affinity column (see step 7) are analysed by 10% SDS-PAGE (Sambrook, *supra*) in two duplicate gels. For one gel, the separated samples are transferred to nitrocellulose by western (see Harlow & Lane, 1988, *Antibodies* (1st ed.) Cold Spring Harbor Laboratory, Cold Spring Harbour, New York). The presence of the fusion protein is determined by immublotting with murine anti human caspase-3 (Roche diagnostics) or murine anti-polyhistidine (Qiagen), followed by anti-mouse horse radish peroxidase (Harlow & Lane, *supra*). Positive signals are visualised by ECL (Amersham-Pharmacia Biotech). For the second gel, protein is visualised directly by coomassie blue staining (Sigma). Samples containing pure fusion protein are pooled and dialysed against phosphate buffered saline 2 x 5 L, 16h at 4°C.
11. The dialysed protein from above is concentrated by ultrafiltration to 1 mg/ml and tested for activity using peptide substrates, specific for caspase-3. Peptides such as the fluorogenic-DEVD based peptides (Calbiochem) can be used in phosphate buffered saline, pH 7.4. Activity is monitored by following the increase in fluorescence and comparison with pure recombinant human caspase 3, either produced as described above or purchased from Calbiochem. Total protein content can be measured by the Bradford assay (Sambrook, *supra).*
12.The antigen binding activity of the fusion protein is measured by ELISA, using mucin-expressing tumour cells (*e.g.* MCF-7 cell line), fixed on 96-well plates with glutaraldehyde (see Harlow & Lane, *supra*).

### (C) Assessment of antigen (i.e. PEM)-mediated delivery of caspase-3 in the mouse model

*In vivo* delivery of caspase is assessed in nude mice (bred in-house at the centre for biomedical studies (CBS), Imperial College, London). Nude mice are used when about 2 weeks old and have subcutaneously grafted tumours, growing for 2 weeks (see Deonarain *et al*., 1997, *Protein Engin.* 10, 89-98). The tumour cell line used is MCF-7. The HuHMFG-1 Fab-Caspase fusion protein (10-100µg in 100µl is injected intravenously via the tail vein of the mouse. The dose is repeated every 3-5 days and tumour regression is assessed by plotting tumour volume, using calipers to measure the tumour dimensions in 3 planes, against time.

### (D) Use of the caspase-3/huHMFG-1 Fab fusion protein in the treatment of ovarian cancer

Patients diagnosed with ovarian cancer are treated by intravenous injection of the caspase-3/huHMFG-1 Fab fusion protein. Typically, a dose of between 1 to 100 mg will be administered weekly.

Therapeutic response is measured by the normal clinical procedures that are well-known in the art, for example radio-imaging methods.

### (E) Construction of a vector to express a scFv-rearranged caspase 3 fusion protein

1. The Caspase-3 gene is used as a template to amplify (by PCR) the small subunit sub-gene using primers CASP3SMFW AND CASP3SMBK (see Table 4), which contains flanking *Bam* H I and *Sal* I restriction sites (Fig. 4, reaction A).
2. The Caspase-3 gene is used as a template to amplify (by PCR) the large subunit sub-gene using primers CASP3LGFW AND CASP3LGBK (see Table 4), which contains flanking *Nco* I and *Bam* HI restriction sites (Fig. 4, reaction B).
3. The large subunit subgene PCR product is digested with NcoI/BamHI restriction enzymes and ligated into the *Nco* I/*Bam HI* sites of pET20b expression vector (Novagen; Fig. 4, reaction C). The resulting plasmid is called pET20LCasp3. The small subunit subgene PCR product is digested and ligated into the *Bam* HI/*Sal* I sites of the pET20LCasp3 plasmid to form the plasmid pET20LSCasp3 (Fig. 4, reaction C). This construct can be used to express the active caspase-3 alone, in *E. coli*, to test for activity and characterise the recombinant enzyme.
4. A vector containing the MFE-23 scFv, pUC119MFE (R. Begent, Royal Free Hospital, London) is used as a source of scFv. The scFv is amplified as a Nco I cassette using the primers MFENCOFOR and MFENCOBAC (see Table 5) (Fig. 4, reaction D). The scFv gene is ligated into the Nco I site of the pET20LSCasp3 to form the vector expressing the scFv-rearranged caspase 3 fusion gene , pET20scFvCasp3 (Fig. 4, reaction E). The DNA sequence of the whole scFv-rearranged caspase 3 gene fusion is described in Figure 5. 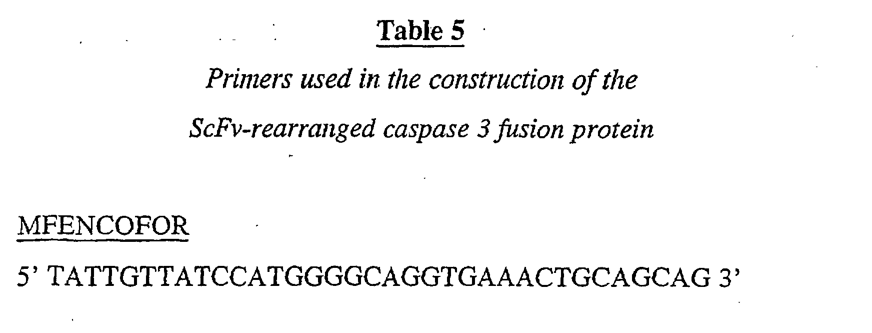 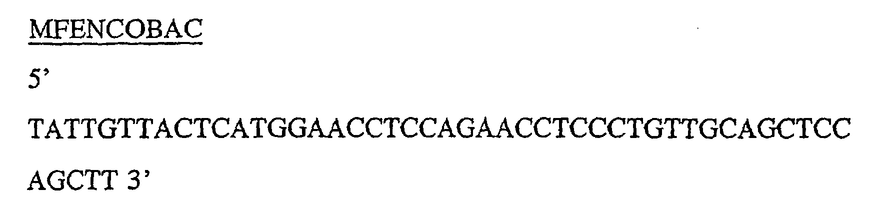
5. Alternatively, the fusion protein can be constructed following the identical scheme (steps 1 to 4), but using the vector pET21d, for cytosolic expression. The *E. coli* strain used is BL21trxb (Novagen) and expression is as described below and modified as described in Linardou et al (2000, *Int. J. Cancer* **86,** 561-569).

### (F) Expression and purification of the scFv-rearranged caspase 3 fusion protein

1. The plasmid pET20scFvCasp3 is transformed into *E. coli* BL21(λDE3)pLysE (Novagen), using the calcium chloride chemical method (Sambrook *et al*, 1989, *Molecular Cloning: A Laboratory Manual* (2nd ed.) Cold Spring Harbor Laboratory, Cold Spring Harbour, New York). Transformants are plated out on 2TYagar plates (Sambrook *et al,* 1989, *Molecular Cloning: A Laboratory Manual* (2nd ed.) Cold Spring Harbor Laboratory, Cold Spring Harbour, New York) containing 100µg/ml ampicillin and 35 µg/ml chloramphenicol and grown overnight at 37°C. Colonies are picked and re-streaked on a fresh plate.
2. A fresh single colony is picked and grown in 5 ml 2TY media with 100µg/ml ampicillin and 35µg/ml chloramphenical for 16 h, shaking at 37°C. This 5 ml starter culture is used to innoculate 1 litre of the same media and grown (shaking at 30°C) until the culture density, as measured by absorbance at 600 nm, is between 0.8 and 1.0. To the culture, 10 ml of 100 mM IPTG (filter sterilised, Sigma) is added and the expression of the fusion protein is induced. The culture continues to grow under the same conditions for 16 h. Figure 6 shows a western blot of the expression of the scFv-caspase protein (detected using anti-His tag antibody, Qiagen). It is produced in the cell pellet (P) and supernatant (SN) after 4 hrs and is present in significant amounts (about 1 mg/L) after 16 hrs. It is the correct molecular weight (about 62 KDa as predicted from the amino acid sequence).
3. Cells are harvested by centrifugation at 10,000 g, 4 °C. The supernatant is retained, concentrated by ultrafiltration in an Amicon cell with a 30 kDa cutoff membrane, to a final volume of 10 ml. The concentrated supernatant is dialysed against phosphate buffered saline containing 2 mM MgCl₂ and 1 M NaCl. The dialysis involves 5-6 changes of 5 litres, for 3-4 h each at 4°C.
4. The scFv-caspase fusion protein is purified from the culture supernatant by metal affinity chromatography on a copper (II) chelating sepharose column (Amersham-Pharmacia Biotech). The samples are pooled and loaded in PBS/1 M NaCl (column buffer) onto about 5 ml of charged resin. The column is washed with 100 ml of column buffer. Next, a 0-200 mM imidazole gradient is applied over a total volume of 200 ml. The fusion protein elutes between 50-100 mM imidazole.

### (G) Characterisation of the scFv-rearranged caspase 3 fusion protein

1. The pure fusion protein from (F) above is concentrated by ultrafiltration to 1 mg/ml, dialysed as above and tested for activity using peptide substrates, specific for caspase-3. Peptides such as the fluorogenic-DEVD based peptides (Calbiochem) can be used in phosphate buffered saline, pH 7.4. Activity is monitored by following the increase in fluorescence and comparison with pure recombinant human caspase 3, either produced as described above or purchased from Calbiochem. Total protein content can be measured by the Bradford assay (Sambrook et al, 1989, *Molecular Cloning: A Laboratory Manual* (2nd ed.) Cold Spring Harbor Laboratory, Cold Spring Harbour, New York).
2. The antigen binding activity of the fusion protein was measured by ELISA (Harlow & Lane, 1988, *Antibodies.* 1st ed. Cold Spring Harbor Laboratory, Cold Spring Harbour, New York) using pure CEA immobilised on ELISA plates. Figure 7 shows some ELISA results of two samples of 1µg/ml protein. These show that the scFv-caspase fusion protein binds to CEA as well as the scFv alone.
4. The bacterially expressed rearranged caspase 3 and scFv-rearranged caspase 3 fusion protein did not cleave the peptide substrate analogue (Ac-DEVD-AMC, Calbiochem), as does the wild-type caspase 3. This has also been noted by others (Prof. N. Lemoine, ICRF, unpublished work). This may be because the rearranged caspase has a subtly altered substrate specificity. The rearranged caspase-3 is cytotoxic to mammalian cells when over-expressed by gene delivery (Prof. N. Lemoine, ICRF, unpublished work), suggesting that the *in vivo* substrate specificity is unaffected. Indeed this is shown to be true when the scFv-rearranged caspase-3 protein is incubated with human cell lysates containing poly-ADP ribose binding protein (PARP), a natural substrate for caspase 3. Figure 8 shows that PARP is cleaved by the scFv- rearranged caspase 3 fusion protein (lane 2), as does pure caspase 3 (lane 4).

### (H) Cytotoxicity of scFv-rearranged caspase 3 fusion protein

A 96 well microtitre plate was seeded with 10⁴ CEA-expressing cells (LS174T) in DMEM media supplemented with 10% serum and grown overnight at 37 °C in a 5% CO₂ humidified incubator. A similar plate was seeded with a CEA negative cell line (KB) and grown under the same conditions. Each well was treated in quadruplicate with bacterial supernatant, concentrated 5-fold after expression of either the scFv alone or the scFv-caspase fusion protein. Seven scFv-caspase clones were picked, which had varying levels of expression of the recombinant protein. Only one scFv clone was tested as this has already been well characterised and is known not to be cytotoxic to these cell lines. Fifty microlitres or ten microlitres of sample was added to each well containing the target cells in 200 µl of complete media. The scFv and scFv-caspase fusion proteins were incubated with the cells for 48 hr and cell death was measured using the cytotox-96 kit (Promega). Controls used were untreated cells and untreated cells undergoing total cell lysis.

It was observed (Figure 9) that the 2 concentrations of scFv alone were not cytotoxic to the CEA-positive cells. All seven scFv-caspase clones tested were cytotoxic to the LS174T cells at the low (10µl) and higher (50µl) doses. Clone 6 was particularly potent demonstrating approximately 75% cell killing in 48 hrs. There was no effect on the negative cell line (KB) and all the standard errors were less than 1.5%.

Even though CEA does not internalise effectively, these results indicate that the rearranged, active caspase 3, when targeted to tumour cells with a recombinant antibody is cytotoxic. The skilled person will therefore appreciate that using a rearranged active caspase 3 fusion protein that binds to more readily internalised targets, for example an HMFG-1 fusion protein, will have even greater cytotoxicity.

## Claims

1. A compound comprising a target cell-specific portion and a cytotoxic portion, **characterised in that** the cytotoxic second portion is a constitutively active caspase or has substantially the same apoptosis-inducing activity as said caspases without the need for activation by other components of the apoptotic cascade as herein before described.

2. A compound comprising a mediator portion capable of recognising a target cell-specific molecule and a cytotoxic second portion, **characterised in that** the cytotoxic second portion is a constitutively active caspase or has substantially the same apoptosis-inducing activity as said caspases without the need for activation by other components of the apoptotic cascade as herein before described.

3. A compound according to Claim 1 wherein the target cell-specific portion recognises and selectively binds to a tumour cell antigen.

4. A compound according to Claim 2 wherein the mediator portion recognises a compound according to Claim 1.

5. A compound according to Claim 2 or 4 wherein the target cell-specific molecule recognised by the mediator portion is derivatised.

6. A compound as claimed in any preceding claim wherein the target cell-specific portion or the mediator portion is an antibody or an antigen binding fragment thereof.

7. A compound as claimed in any preceding claim wherein the target cell-specific portion is internalised upon contact with the target cell.

8. A compound as claimed in any preceding claim wherein the target cell-specific portion or the mediator portion is an HMFG-1 antibody or an antigen binding fragment thereof.

9. A compound according to any of claims 6 to 8 wherein the antibody or antigen binding fragment thereof is humanised.

10. A compound according to any preceding claim wherein the cytotoxic portion is at least the catalytically active portion of a constitutively active caspase.

11. A compound according to any preceding claim wherein the cytotoxic portion is a constitutively active effector caspase or has substantially the same apoptosis-inducing activity as the said caspase without the need for activation by other components of the apoptotic cascade as herein before described.

12. A compound according to any preceding claim wherein the cytotoxic portion is a constitutively active caspase-3, caspase 6 or caspase 7, or has substantially the same apoptosis-inducing activity as the said caspase without the need for activation by other components of the apoptotic cascade as herein before described.

13. A compound according to any preceding claim wherein the cytotoxic portion is of mammalian origin.

14. A compound according to any preceding claim wherein the cytotoxic portion is constitutively active variant of a naturally occurring caspase.

15. A compound according to any preceding claim wherein the cytotoxic portion is capable of oligomerisation.

16. A compound according to any preceding claim wherein said compound is a fusion compound.

17. An isolated nucleic acid molecule encoding a compound according to any one of Claims 1 to 16.

18. A method of making a compound according to any one of Claims 1 to 16, said method comprising expressing one or more nucleic acid molecules according to Claim 17 in a host cell and isolating the compound therefrom.

19. A vector for expressing in a host cell a compound according to any one of Claim 1 to 16 or a portion thereof, said vector comprising one or more nucleic acid molecules according to Claim 17.

20. A host cell transformed with a vector according to Claim 19.

21. A pharmaceutical composition comprising a compound according to any one of Claims 1 to 16 and a pharmaceutically acceptable carrier or excipient.

22. A compound according to any one of Claims 1 to 16 for use in medicine.

23. Use of a compound according to any one of Claims 1 to 16 in the preparation of a medicament for treating a disease associated with the dysfunction of a population of cells.

24. The use according to Claim 23 for treating cancer.

## Patentansprüche

1. Verbindung, die einen targetzellspezifischen Teil und einen cytotoxischen Teil umfasst, **dadurch gekennzeichnet, dass** der cytotoxische zweite Teil eine konstitutiv aktive Caspase ist oder im wesentlichen die gleiche apoptoseinduzierende Aktivität wie die Caspasen ohne die Notwendigkeit der Aktivierung durch andere Komponenten der Apoptosekaskade.

2. Verbindung, die einen Vermittlerteil mit der Fähigkeit zum Erkennen eines targetzellspezifischen Moleküls und einen cytotoxischen zweiten Teil umfasst, **dadurch gekennzeichnet, dass** der cytotoxische zweite Teil eine konstitutiv aktive Caspase ist oder im wesentlichen die gleiche apoptoseinduzierende Aktivität wie die Caspasen ohne die Notwendigkeit der Aktivierung durch andere Komponenten der Apoptosekaskade.

3. Verbindung nach Anspruch 1, wobei der targetzellspezifische Teil ein Tumorzellantigen erkennt und selektiv an dieses bindet.

4. Verbindung nach Anspruch 2, wobei der Vermittlerteil eine Verbindung nach Anspruch 1 erkennt.

5. Verbindung nach Anspruch 2 oder 4, wobei das durch den Vermittlerteil erkannte targetzellspezifische Molekül derivatisiert ist.

6. Verbindung nach einem der vorhergehenden Ansprüche, wobei der targetzellspezifische Teil oder der Vermittlerteil ein Antikörper oder ein antigenbindendes Fragment desselben ist.

7. Verbindung nach einem der vorhergehenden Ansprüche, wobei der targetzellspezifische Teil bei Kontakt mit der Targetzelle internalisiert bzw. in die Zelle aufgenommen wird.

8. Verbindung nach einem der vorhergehenden Ansprüche, wobei der targetzellspezifische Teil oder der Vermittlerteil ein HMFG-1-Antikörper oder ein antigenbindendes Fragment desselben ist.

9. Verbindung nach einem der Ansprüche 6 bis 8, wobei der Antikörper oder das antigenbindende Fragment desselben humanisiert ist.

10. Verbindung nach einem der vorhergehenden Ansprüche, wobei der cytotoxische Teil mindestens der katalytisch aktive Teil einer konstitutiv aktiven Caspase ist.

11. Verbindung nach einem der vorhergehenden Ansprüche, wobei der cytotoxische Teil eine konstitutiv aktive Effektorcaspase ist oder im wesentlichen die gleiche apoptoseinduzierende Aktivität wie die Caspase ohne die Notwendigkeit der Aktivierung durch andere Komponenten der Apoptosekaskade.

12. Verbindung nach einem der vorhergehenden Ansprüche, wobei der cytotoxische Teil eine konstitutiv aktive Caspase 3, Caspase 6 oder Caspase 7 ist oder im wesentlichen die gleiche apoptoseinduzierende Aktivität wie die Caspase ohne die Notwendigkeit der Aktivierung durch andere Komponenten der Apoptosekaskade.

13. Verbindung nach einem der vorhergehenden Ansprüche, wobei der cytotoxische Teil von Säugetieren stammt.

14. Verbindung nach einem der vorhergehenden Ansprüche, wobei der cytotoxische Teil eine konstitutiv aktive Variante einer natürlich vorkommenden Caspase ist.

15. Verbindung nach einem der vorhergehenden Ansprüche, wobei der cytotoxische Teil zur Oligomerisierung fähig ist.

16. Verbindung nach einem der vorhergehenden Ansprüche, wobei die Verbindung eine Fusionsverbindung ist.

17. Isoliertes Nucleinsäuremolekül mit Codierung für eine Verbindung nach einem der Ansprüche 1 bis 16.

18. Verfahren zur Herstellung einer Verbindung nach einem der Ansprüche 1 bis 16, wobei das Verfahren die Expression von einem oder mehreren Nucleinsäuremolekülen nach Anspruch 17 in einer Wirtszelle und das Isolieren der Verbindung aus dieser umfasst.

19. Vektor zur Expression einer Verbindung nach einem der Ansprüche 1 bis 16 oder eines Teils derselben in einer Wirtszelle, wobei der Vektor ein oder mehrere Nucleinsäuremoleküle nach Anspruch 17 umfasst.

20. Wirtszelle, die mit einem Vektor nach Anspruch 19 transformiert wurde.

21. Pharmazeutische Zusammensetzung, die eine Verbindung nach einem der Ansprüche 1 bis 16 und einen pharmazeutisch akzeptablen Träger oder ein pharmazeutisch akzeptables Streckmittel umfasst.

22. Verbindung nach einem der Ansprüche 1 bis 16 zur Verwendung in der Medizin.

23. Verwendung einer Verbindung nach einem der Ansprüche 1 bis 16 bei der Herstellung eines Medikaments zur Behandlung einer Erkrankung, die mit der Dysfunktion einer Zellpopulation verbunden ist.

24. Verwendung nach Anspruch 23 zur Behandlung von Krebs.

## Revendications

1. Composé comprenant une partie spécifique vis-à-vis de cellules cibles et une partie cytotoxique, **caractérisé en ce que** la seconde partie cytotoxique est une caspase active par sa constitution ou bien a substantiellement la même activité d'induction d'apoptose que lesdites caspases sans besoin d'activation par d'autres composants du processus d'apoptose en cascade.

2. Composé comprenant une partie médiatrice ayant la capacité de reconnaître une molécule spécifique vis-à-vis de cellules cibles et une seconde partie cytotoxique, **caractérisé en ce que** la seconde partie cytotoxique est une caspase active par sa constitution ou bien a substantiellement la même activité d'induction d'apoptose que lesdites caspases sans besoin d'activation par d'autres composants du processus d'apoptose en cascade.

3. Composé selon la revendication 1, dans lequel la partie spécifique vis-à-vis de cellules cibles reconnaît et se lie sélectivement à un antigène des cellules tumorales.

4. Composé selon la revendication 2, dans lequel la partie médiatrice reconnaît un composé selon la revendication 1.

5. Composé selon la revendication 2 ou 4, dans lequel la molécule spécifique vis-à-vis de cellules cibles reconnue par la partie médiatrice est dérivée.

6. Composé tel que défini dans l'une quelconque des revendications précédentes, dans lequel la partie spécifique vis-à-vis de cellules cibles ou la partie médiatrice est un anticorps ou un fragment de celui-ci de liaison à l'antigène.

7. Composé tel que défini dans l'une quelconque des revendications précédentes, dans lequel la partie spécifique vis-à-vis de cellules cibles est incluse dès le contact avec la cellule cible.

8. Composé tel que défini dans l'une quelconque des revendications précédentes, dans lequel la partie spécifique vis-à-vis de cellules cibles ou la partie médiatrice est un anticorps HMFG-1 ou un fragment de celui-ci de liaison à l'antigène.

9. Composé selon l'une quelconque des revendications 6 à 8, dans lequel l'anticorps ou le fragment de celui-ci de liaison à l'antigène est rendu humain.

10. Composé selon l'une quelconque des revendications précédentes, dans lequel la partie cytotoxique est au moins la partie catalytiquement active d'une caspase active par sa constitution.

11. Composé selon l'une quelconque des revendications précédentes, dans lequel la partie cytotoxique est une caspase effectrice active par sa constitution ou bien a substantiellement la même activité d'induction d'apoptose que ladite caspase sans besoin d'activation par d'autres composants du processus d'apoptose en cascade.

12. Composé selon l'une quelconque des revendications précédentes, dans lequel la partie cytotoxique est une caspase-3, caspase-6 ou caspase-7 active par sa constitution ou bien a substantiellement la même activité d'induction d'apoptose que ladite caspase sans besoin d'activation par d'autres composants du processus d'apoptose en cascade.

13. Composé selon l'une quelconque des revendications précédentes, dans lequel la partie cytotoxique est d'origine mammifère.

14. Composé selon l'une quelconque des revendications précédentes, dans lequel la partie cytotoxique est une variante active par sa constitution d'une caspase d'origine naturelle.

15. Composé selon l'une quelconque des revendications précédentes, dans lequel la partie cytotoxique est susceptible d'oligomérisation.

16. Composé selon l'une quelconque des revendications précédentes, dans lequel ledit composé est un composé de fusion.

17. Molécule d'acide nucléique isolée codant pour un composé selon l'une quelconque des revendications 1 à 16.

18. Procédé de production d'un composé selon l'une quelconque des revendications 1 à 16, ledit procédé comprenant l'expression d'une ou plusieurs molécules d'acide nucléique selon la revendication 17 dans une cellule hôte et l'isolement du composé à partir de celle-ci.

19. Vecteur d'expression dans une cellule hôte d'un composé selon l'une quelconque des revendications 1 à 16 ou d'une partie de celui-ci, ledit vecteur comprenant une ou plusieurs molécules d'acide nucléique selon la revendication 17.

20. Cellule hôte transformée par un vecteur selon la revendication 19.

21. Composition pharmaceutique comprenant un composé selon l'une quelconque des revendications 1 à 16 et un véhicule ou excipient pharmaceutiquement acceptable.

22. Composé selon l'une quelconque des revendications 1 à 16 pour une utilisation en médecine.

23. Utilisation d'un composé selon l'une quelconque des revendications 1 à 16 dans la préparation d'un médicament pour le traitement d'une maladie associée au disfonctionnement d'une population de cellules.

24. Utilisation selon la revendication 23 pour un traitement du cancer.
